# EUROPEAN PATENT APPLICATION

(11) **EP 1 553 181 A1**
(43) Date of publication of application: **13.07.2005**
(21) Application number: 04290076.1
(22) Date of filing: 12.01.2004
(51) Int. Cl.: C12N 15/11

(54) **Gene expression inhibition and uses thereof**

(71) Applicant: Jais, Philippe, 92130 Issey-les-Moulineaux (FR)
(72) Inventor: Jais, Philippe, 92130 Issey-les-Moulineaux (FR)
(74) Representative: Gallois, Valérie

(57) **Abstract**

The present invention relates to methods and compositions for inhibiting gene expression in a cell or organism, as well as uses thereof. More particularly, the invention is based on the conception and design of selective and efficient inhibitory oligonucleotides that are expressed in cells in the presence of an RNA polymerase. The method thus preferably utilizes a (recombinant) cell or a non-human (transgenic) organism expressing a single-subunit RNA polymerase and a molecule comprising a sequence encoding an expression-inhibiting oligonucleotide specific for a targeted gene operably linked to element(s) allowing transcription of said sequence by the single-subunit RNA polymerase in said cell or organism. Preferably, the expression-inhibiting oligonucleotide transcribed by the cells is an antisense RNA, a siRNA, a shRNA or a ribozyme. The present invention also relates to the use of this gene expression inhibition system for identifying/studying the function of a targeted gene, for validating a therapeutic target, or for optimizing an expression-inhibiting oligonucleotide. The present invention further relates to a kit for implementing this gene expression inhibition system.

SEQUENCE LISTING

<110>  JAIS, Philippe

<120>  Gene expression inhibition and uses thereof

<130>  B0237

<160>  9

<170>  PatentIn version 3.1

<210>  1
<211>  17
<212>  DNA
<213>  Bacteriophage T7

<400>  1
tatagtgagt cgtatta                                                    17

<210>  2
<211>  17
<212>  DNA
<213>  Bacteriophage T3

<400>  2
aattaaccct cactaaa                                                    17

<210>  3
<211>  17
<212>  DNA
<213>  Bacteriophage K11

<400>  3
aattaggcca cactata                                                    17

<210>  4
<211>  17

<212>  DNA
<213>  Bacteriophage SP6

<400>  4
atttaggtga cactata                                                    17

<210>  5
<211>  39
<212>  DNA
<213>  artificial sequence

<220>
<223>  Hairpin T7 mininal promoter
<400>  5
tatagtgagt cgtattatca agtaatacga ctcactata                            39

<210>  6
<211>  51
<212>  DNA
<213>  artificial sequence

<220>
<223>  hairpin extended T7 promoter
<400>  6
tatagtgagt cgtattatgg atcccaaggg atcctaatac gactcactat a              51

<210>  7
<211>  23
<212>  DNA
<213>  Bacteriophage T7

<400>  7
tatagtgagt cgtattagga tcc                                             23

<210>  8
<211>  23
<212>  DNA
<213>  Bacteriophage T7

<400>  8
ggatcctaat acgactcact ata                                                23

<210>  9

<211>  17

<212>  DNA

<213>  Bacteriophage T7

<400>  9
taatacgact cactata                                                       17

## Description

### FIELD OF THE INVENTION

The present invention relates to methods and compositions for inhibiting gene expression in a cell or organism, as well as uses thereof. More particularly, the invention is based on the conception and design of selective and efficient inhibitory oligonucleotides that are expressed in cells in the presence of an RNA polymerase. The method thus preferably utilizes a (recombinant) cell or a non-human (transgenic) organism expressing a single-subunit RNA polymerase and a molecule comprising a sequence encoding an expression-inhibiting oligonucleotide specific for a targeted gene operably linked to element(s) allowing transcription of said sequence by the single-subunit RNA polymerase in said cell or organism. Preferably, the expression-inhibiting oligonucleotide transcribed by the cells is a antisense RNA, a siRNA, a shRNA or a ribozyme. The present invention also relates to the use of this gene expression inhibition system for identifying/studying the function of a targeted gene, for validating a therapeutic target, or for optimizing an expression-inhibiting oligonucleotide. The present invention further relates to a kit for implementing this gene expression inhibition system.

### BACKGROUND OF THE INVENTION

With the completion of genome sequencing of various species, including human and mouse, it is now possible to achieve genome-wide prediction for the presence of genes and their cellular functions. However, such predictions, which are mostly based on bioinformatic analysis, remain to be tested and/or validated by biological methods. Such methods are widely used at the target validation phase of the drug R&D process period. This phase representing a quarter of R&D expenditure required to produce a new drug.

The most frequently used technologies for gene function analysis are based on gene expression or disruption. However, these technologies have serious limitations such as their unsuitability in case of incomplete knowledge of the gene sequence, their high cost, and the time required for completion. An interesting alternative is based on inhibition of gene expression by chemically synthesized oligonucleotides. Several types of oligonucleotides can be used, including antisense oligodeoxynucleotides, ribozymes, short interfering RNA (siRNA) and short hairpin RNA (shRNA). Although very encouraging results have been obtained, especially with ribozymes, shRNA and siRNA, these methods have serious limitations. Firstly, the synthesis of RNA oligonucleotides, i.e. siRNA and ribozymes, is very expensive. Secondly, these RNA oligonucleotides are highly unstable in biological medium and cannot be readily utilized to inhibit gene expression in living organisms. Although ribozymes and siRNA can be protected against nucleolytic attack, chemical modifications frequently alter their catalytic activity and this approach remains hazardous (Clarenc, Lebleu et al. 1993; Gao, Storm et al. 1993; Beltinger, Saragovi et al. 1995; Aoki, Morishita et al. 1997; Oehlke, Birth et al. 2002; Kurreck 2003). Thirdly, the cellular uptake of naked oligonucleotides is quite inefficient (Clarenc, Lebleu et al. 1993; Gao, Storm et al. 1993; Beltinger, Saragovi et al. 1995; Aoki, Morishita et al. 1997; Oehlke, Birth et al. 2002; Kurreck 2003). This can be overcome by lipid-transfection of vectors encoding such oligonucleotides, but this step complicates the use of oligonucleotides and increases the cost of the experiments. Fourthly, most of the synthetic oligonucleotides accumulate in inappropriate compartments within eukaryotic cells, i.e. the liposomes and the nucleus, while they are supposed to act in the cytoplasm (Clarenc, Lebleu et al. 1993; Gao, Storm et al. 1993; Beltinger, Saragovi et al. 1995; Aoki, Morishita et al. 1997; Oehlke, Birth et al. 2002; Kurreck 2003). Fifthly, synthetic oligonucleotides have a poor affinity towards the target mRNA. Consequently, DNA:RNA duplexes are rather unstable, which likely hamper their efficacy (Kurreck 2003). This explains, at least in part, the inefficiency of certain types of oligonucleotides.

### SUMMARY OF THE INVENTION

The present invention provides improved methods and compositions for oligonucleotide-mediated selective gene expression inhibition, particularly antisense oligonucleotides, ribozymes and siRNA. The invention is based on the design of particular molecule that causes efficient production of biologically active oligonucleotides in a cell or an organism expressing a RNA polymerase. In this regard, the present invention typically requires: 1°) A cell or a non-human organism expressing a single-subunit RNA polymerase; and, 2°) at least one molecule comprising a sequence encoding an expression-inhibiting oligonucleotide specific for a targeted gene, operably linked to element(s) allowing transcription of said sequence by the single-subunit RNA polymerase in the cell or the non-human organism. Depending on the design of the molecules, the cells or organisms can produce, upon transcription of said sequence, siRNA, shRNA, antisense RNA or ribozymes, resulting in high cellular levels of siRNA, shRNA, antisense RNA or ribozymes which cause a selective and efficient inhibition of the expression of the targeted gene in the cell or in the non-human organism.

In a particular aspect, the present invention concerns a method of inhibiting the expression of a targeted gene in a cell or a non-human organism expressing a single-subunit RNA polymerase, comprising the following steps:
- providing a molecule comprising a sequence encoding an expression-inhibiting oligonucleotide specific for said targeted gene, operably linked to element(s) allowing transcription of said sequence by the single-subunit RNA polymerase in the cell or the non-human organism;
- introducing said molecule into said cell or non-human organism so that said inhibiting-sequence is expressed in said cell or non-human organism, thereby inhibiting the expression of said targeted gene.

In an additional aspect, the present invention concerns a method of inhibiting the expression of a targeted gene in a cell or a non-human organism, wherein said cell or non-human organism comprises i) a single-subunit RNA polymerase gene capable of being expressed as an RNA polymerase protein in said cell or organism and ii) a molecule comprising a sequence encoding an expression-inhibiting oligonucleotide specific for said targeted gene, operably linked to element(s) allowing transcription of said sequence by the single-subunit RNA polymerase in said cell or organism, comprising the step of :
- a) culturing said cell or organism under condition whereby a sufficient quantity of said RNA polymerase protein is expressed to cause the transcription of said expression-inhibiting oligonucleotide;
- b) observing the inhibition of the expression of said targeted gene.

In an other aspect, the present invention also concerns a method for identifying or studying the function of a targeted gene in a cell or a non-human organism, wherein said cell or non-human organism comprises i) a single-subunit RNA polymerase gene capable of being expressed as an RNA polymerase protein in said cell or organism and ii) a molecule comprising a sequence encoding an expression-inhibiting oligonucleotide specific for said targeted gene, operably linked to element(s) allowing transcription of said sequence by the single-subunit RNA polymerase in said cell or organism, comprising the step of:
- a) culturing said cell or organism under condition whereby a sufficient quantity of said RNA polymerase protein is expressed to cause the transcription of said expression-inhibiting oligonucleotide;
- b) determining the effect of the inhibition of expression of said gene on said cell or organism, thereby identifying or studying the function of a gene.

In a further aspect, the present invention also concerns a method for identifying or studying the function of a gene in a cell or a non-human organism expressing a single-subunit RNA polymerase, comprising the following steps:
- providing a molecule comprising a sequence encoding an expression-inhibiting oligonucleotide specific for said gene, operably linked to element(s) allowing transcription of said sequence by the single-subunit RNA polymerase in the cell or the non-human organism;
- introducing said molecule into said cell or non-human organism so that said sequence is expressed in said cell or non-human organism, thereby inhibiting the expression of said targeted gene; and,
- determining the effect of the inhibition of expression of said gene on said cell or organism, thereby identifying or studying the function of a gene.

Optionally, the determination of the effect of the inhibition of expression of said gene on said cell or organism is performed by the observation of the phenotype of cell or organism.

The present invention further concerns a method for optimizing an expression-inhibiting oligonucleotide specific for a targeted gene in a cell or a non-human organism expressing a single-subunit RNA polymerase, comprising the following step:
- providing a first molecule comprising a sequence encoding a first expression-inhibiting oligonucleotide specific for said targeted gene, operably linked to element(s) allowing transcription of said sequence by the single-subunit RNA polymerase in the cell or the non-human organism;
- providing a second molecule comprising a sequence encoding a second expression-inhibiting oligonucleotide specific for said targeted gene, operably linked to element(s) allowing transcription of said sequence by the single-subunit RNA polymerase in the cell or the non-human organism;
- introducing said first molecule into said cell or non-human organism so that said sequence is expressed in said cell or non-human organism, thereby inhibiting the expression of said targeted gene;
- introducing said second molecule into an other said cell or non-human organism so that said sequence is expressed in said cell or non-human organism, thereby inhibiting the expression of said targeted gene; and,
- determining and comparing the inhibition of expression of said targeted gene on said cell or organism with either a first molecule or a second molecule.

The cell or organism may naturally express the selected RNA polymerase, or they can be engineered to produce the same recombinantly. In a preferred embodiment, the methods comprise an initial step of preparing or providing a recombinant cell or a non-human transgenic organism expressing a single-subunit RNA polymerase.

A further aspect of the present invention relates to a method for preparing a cell or a non-human organism in which the expression of a targeted gene is inhibited, comprising the following steps:
- providing a cell or a non-human organism expressing a single-subunit RNA polymerase;
- providing at least one molecule comprising a sequence encoding an expression-inhibiting oligonucleotide specific for said targeted gene, operably linked to element(s) allowing transcription of said sequence by the single-subunit RNA polymerase in the cell or the organism;
- introducing said molecule into said cell or non-human organism so that said sequence is expressed in said cell or non-human organism, thereby inhibiting the expression of said targeted gene.

The invention also relates to a methof of preparing a non-human organism with an inhibited expression of a targeted gene, comprising the steps of:
a) introducing into said organism a single subunit RNA polymerase gene capable of being expressed as an RNA polymerase protein in said organism; and,
b) introducing into said organism a molecule comprising a sequence encoding an expression-inhibiting oligonucleotide specific for said targeted gene, operably linked to element(s) allowing transcription of said sequence by said single-subunit RNA polymerase in the organism.
Optionally, process step a) can occur prior to step b). Alternatively, process step b) can occur prior to step a).

In a preferred embodiment of the methods according to the present invention, the single-subunit RNA polymerase is a bacteriophage RNA polymerase, more preferably selected from the group consisting of T7 RNA polymerase, T3 RNA polymerase, SP6 RNA polymerase, and K11 RNA polymerase, still more preferably T7 RNA polymerase or T3 RNA polymerase, even more preferably T7 RNA polymerase. Alternatively, the single-subunit RNA polymerase can be a mitochondrial RNA polymerase. The single-subunit RNA polymerase can also be a chloroplast RNA polymerase. In a preferred embodiment of the methods according to the present invention, the targeted gene is a genomic gene. In a preferred embodiment of the methods according to the present invention, the expression-inhibiting oligonucleotide is selected from the group consisting of an antisense RNA, a siRNA, a shRNA and a ribozyme. In a preferred embodiment of the methods according to the present invention, said element(s) allowing transcription of said sequence comprises the minimal promoter of said single-subunit RNA polymerase, more preferably the promoter sequence of nucleotides between positions -17 and +1, -17 and +2, -23 and +1 or -23 and +2 of the single subunit RNA polymerase (nucleotide +1 corresponds to the transcription start), more preferably the promoter sequence of nucleotides between positions -17 and +1 of the single subunit RNA polymerase. Optionally, said element(s) allowing transcription of said sequence comprises a terminator of said single-subunit RNA polymerase. In a preferred embodiment of the methods according to the present invention, the cell is a eukaryotic cell, preferably a mammalian cell, more preferably a human or a mouse cell. In a preferred embodiment of the methods according to the present invention, the non-human organism is a non-human mammalian, preferably a mouse, a pig, a rabbit, or a rat, more preferably a mouse. Preferably, the expression of said single-subunit RNA polymerase is constitutive. Alternatively, the expression of said single-subunit RNA polymerase can be regulated (e.g., inducible). The expression of said single-subunit RNA polymerase may also be ubiquitous or tissue-specific. In a particular embodiment of the methods according to the present invention, said cell or said non-human organism expresses two different single subunit RNA polymerases. In this particular embodiment, at least two different molecules comprising a sequence encoding an expression-inhibiting oligonucleotide are used, each molecule comprising a sequence encoding an expression-inhibiting oligonucleotide operably linked to element(s) allowing transcription by one of the RNA polymerases. This embodiment is particularly useful when the expression-inhibiting oligonucleotide is a siRNA, wherein the first strand of the siRNA encoded by a first molecule under the control of a first RNA polymerase promoter, and the second strand of the siRNA is encoded by a second molecule under the control of a second RNA polymerase promoter.

The present invention also concerns a kit for implementing a method as disclosed therein. Typically, such a kit comprises a cell or a non-human organism expressing a single-subunit RNA polymerase and at least one molecule comprising a sequence encoding an expression-inhibiting oligonucleotide specific for a targeted gene, operably linked to element(s) allowing transcription of said sequence by the single-subunit RNA polymerase in said cell or said transgenic organism. Alternatively, such a kit can comprises at least one molecule comprising a sequence encoding an expression-inhibiting oligonucleotide specific for a targeted gene, operably linked to element(s) allowing transcription of said sequence by a single-subunit RNA polymerase and instructions on how to use it with a cell or a non-human organism expressing the single-subunit RNA polymerase. Preferably, the single-subunit RNA polymerase is a bacteriophage RNA polymerase, more preferably selected from the group consisting of T7 RNA polymerase, T3 RNA polymerase, SP6 RNA polymerase, and K11 RNA polymerase, still more preferably is T7 RNA polymerase or T3 RNA polymerase, even more preferably T7 RNA polymerase. Alternatively, the single-subunit RNA polymerase is a mitochondrial or a chloroplast RNA polymerase. In a preferred embodiment, said non-human organism is a non-human mammal, more preferably a mouse, a rabbit, a rat or a pig, still more preferably a mouse. In another preferred embodiment, said cell is a eukaryotic cell, more preferably a mammalian cell, still more preferably a human or mouse cell. Optionally, the expression of said single-subunit RNA polymerase is constitutive. Alternatively, the expression of said single-subunit RNA polymerase can be regulated (e.g., inducible). The expression of said single-subunit RNA polymerase may also be ubiquitous or tissue-specific. Preferably, the expression-inhibiting oligonucleotide is selected from the group consisting of an antisense RNA, a siRNA, a shRNA and a ribozyme. Preferably, the element(s) allowing transcription of said sequence comprises the minimal promoter of said single-subunit RNA polymerase, more preferably the promoter sequence of nucleotides between positions -17 and +1, -17 and +2, -23 and +1 or -23 and +2 of the single subunit RNA polymerase (nucleotide +1 corresponds to the transcription start), more preferably the promoter sequence of nucleotides between positions -17 and +1 or -23 and +1 of the single subunit RNA polymerase. Optionally, the element(s) allowing transcription of said sequence comprises a terminator of said single-subunit RNA polymerase. In a particular embodiment of the kit according to the present invention, said cell or said non-human organism expresses two different single subunit RNA polymerases. In this particular embodiment, at least two different molecules comprising a sequence encoding an expression-inhibiting oligonucleotide are used, each molecule comprising a sequence encoding an expression-inhibiting oligonucleotide operably linked to element(s) allowing transcription by one of the RNA polymerases. This embodiment is particularly useful when the expression-inhibiting oligonucleotide is a siRNA, wherein the first strand of the siRNA is encoded by a first molecule under the control of a first RNA polymerase promoter, and the second strand of the siRNA is encoded by a second molecule under the control of a second RNA polymerase promoter.

The present invention also concerns the use of a non-human organism expressing a single-subunit RNA polymerase in combination with at least one molecule comprising a sequence encoding an expression-inhibiting oligonucleotide specific for a targeted gene, operably linked to element(s) allowing transcription of said sequence by the single-subunit RNA polymerase for inhibiting the expression of said targeted gene, for identifying/studying the function of the targeted gene, for validating a therapeutic target or for optimizing siRNA, shRNA, antisense RNA or ribozymes. Preferably, said organism is a non-human transgenic organism. Preferably, the single-subunit RNA polymerase is a bacteriophage RNA polymerase, more preferably selected from the group consisting of T7 RNA polymerase, T3 RNA polymerase, SP6 RNA polymerase, and K11 RNA polymerase, still more preferably is T7 RNA polymerase or T3 RNA polymerase, even more preferably T7 RNA polymerase. Alternatively, the single-subunit RNA polymerase is a mitochondrial or a chloroplast RNA polymerase. In a preferred embodiment, said non-human organism is a mammal, more preferably a mouse, a rabbit, a rat or a pig, still more preferably a mouse. In another preferred embodiment, said cell is a eukaryotic cell, more preferably a mammalian cell, still more preferably a human cell. Optionally, the expression of said single-subunit RNA polymerase is constitutive. Alternatively, the expression of said single-subunit RNA polymerase can be regulated (e.g., inducible). The expression of said single-subunit RNA polymerase may also be ubiquitous or tissue-specific. Preferably, the expression-inhibiting oligonucleotide is selected from the group consisting of an antisense RNA, a siRNA, a shRNA and a ribozyme. Preferably, the element(s) allowing transcription of said sequence comprises the minimal promoter of said single-subunit RNA polymerase, more preferably the promoter sequence of nucleotides between positions -17 and +1, -17 and +2, -23 and +1 or -23 and +2 of the single subunit RNA polymerase, more preferably the promoter sequence of nucleotides between positions -17 and +1 of the single subunit RNA polymerase. Optionally, the element(s) allowing transcription of said sequence comprises a terminator of said single-subunit RNA polymerase. In a particular embodiment of the use according to the present invention, said cell or said non-human organism expresses two different single subunit RNA polymerases. In this particular embodiment, at least two different molecules comprising a sequence encoding an expression-inhibiting oligonucleotide are used, each molecule comprising a sequence encoding an expression-inhibiting oligonucleotide operably linked to element(s) allowing transcription by one of the RNA polymerases. This embodiment is particularly useful when the expression-inhibiting oligonucleotide is a siRNA, wherein the first strand of the siRNA is encoded by a first molecule under control of a first RNA polymerase promoter, and the second strand of the siRNA is encoded by a second molecule under control of a second RNA polymerase promoter.

The present invention concerns a non-human transgenic mammal expressing a single-subunit RNA polymerase, wherein an expression cassette encoding said single-subunit RNA polymerase is integrated in the genome of said non-human transgenic mammal. Preferably, the single-subunit RNA polymerase is a heterologous single-subunit RNA polymerase. Preferably, the single-subunit RNA polymerase is a bacteriophage RNA polymerase, more preferably selected from the group consisting of T7 RNA polymerase, T3 RNA polymerase, SP6 RNA polymerase, and K11 RNA polymerase, still more preferably is T7 RNA polymerase or T3 RNA polymerase, even more preferably T7 RNA polymerase. Alternatively, the single-subunit RNA polymerase is a mitochondrial or a chloroplast RNA polymerase. In a preferred embodiment, said mammal is a mouse, a rabbit, a rat or a pig. In a more preferred embodiment, said mammal is a mouse. Optionally, the expression of said single-subunit RNA polymerase is constitutive. Alternatively, the expression of said single-subunit RNA polymerase can be regulated (e.g., inducible). The expression of said single-subunit RNA polymerase may also be ubiquitous or tissue-specific. The invention also concerns the use of said non-human transgenic mammal for inhibiting the expression of a targeted gene, for identifying/studying the function of the targeted gene, for validating a therapeutic target or for optimizing siRNA, shRNA, antisense RNA or ribozymes. In a particular embodiment, said non-human transgenic mammal expresses two different single subunit RNA polymerases.

The present invention also concerns a cell or a non-human organism comprising: a) a single subunit RNA polymerase gene capable of being expressed as an RNA polymerase protein in said cell or organism; and, b) a molecule comprising a sequence encoding an expression-inhibiting oligonucleotide specific for a targeted gene, operably linked to element(s) allowing transcription of said sequence by the single-subunit RNA polymerase. Preferably, the present invention concerns a non-human organism.

The present invention also concerns a molecule comprising a sequence encoding an expression-inhibiting oligonucleotide specific for a targeted gene, operably linked to element(s) allowing transcription of said sequence by a single-subunit RNA polymerase. Preferably, the single-subunit RNA polymerase is a bacteriophage RNA polymerase, more preferably selected from the group consisting of T7 RNA polymerase, T3 RNA polymerase, SP6 RNA polymerase, and K11 RNA polymerase, still more preferably is T7 RNA polymerase or T3 RNA polymerase, even more preferably T7 RNA polymerase. Alternatively, the single-subunit RNA polymerase is a mitochondrial or a chloroplast RNA polymerase. Preferably, the expression-inhibiting oligonucleotide is selected from the group consisting of an antisense RNA, a siRNA, a shRNA and a ribozyme. Preferably, the element(s) allowing transcription of said sequence comprises the minimal promoter of said single-subunit RNA polymerase, more preferably the promoter sequence of nucleotides between positions -17 and +1, -17 and +2, -23 and +1 or -23 and +2 of the single subunit RNA polymerase, more preferably the promoter sequence of nucleotides between positions -17 and +1 of the single subunit RNA polymerase. Optionally, the element(s) allowing transcription of said sequence comprises a terminator of said single-subunit RNA polymerase. More particularly, the molecule comprising the sequence encoding the expression-inhibiting oligonucleotide has a structure selected from the group consisting of:
- a hairpin oligonucleotide comprising (or consisting of) a minimal double-stranded RNA polymerase promoter sequence operably linked to a single-stranded oligonucleotide comprising (or consisting of) the sequence encoding the expression-inhibiting oligonucleotide;
- a hairpin oligonucleotide comprising (or consisting of) a minimal double-stranded RNA polymerase promoter sequence operably linked to a double-stranded oligonucleotide comprising (or consisting of) the sequence encoding the expression-inhibiting oligonucleotide;
- an oligonucleotide comprising (or consisting of) a minimal double-stranded RNA polymerase promoter sequence operably linked to a single-stranded oligonucleotide comprising (or consisting of) the sequence encoding the expression-inhibiting oligonucleotide; and,
- an oligonucleotide comprising (or consisting of) a minimal double-stranded RNA polymerase promoter sequence operably linked to a double-stranded oligonucleotide comprising (or consisting of) the sequence encoding the expression-inhibiting oligonucleotide.

More preferably, the molecule comprising the sequence encoding the expression-inhibiting oligonucleotide has a structure selected from the group consisting of:
- a hairpin oligonucleotide comprising (or consisting of) a minimal double-stranded RNA polymerase promoter sequence operably linked to a single-stranded oligonucleotide comprising (or consisting of) the sequence encoding the expression-inhibiting oligonucleotide; and,
- a hairpin oligonucleotide comprising (or consisting of) a minimal double-stranded RNA polymerase promoter sequence operably linked to a double-stranded oligonucleotide comprising (or consisting of) the sequence encoding the expression-inhibiting oligonucleotide.

The invention also relates to the use of a cell or a non-human organism in which the expression of a targeted gene is inhibited according to the present invention for drug screening, cellular therapy, pharmacogenomics, etc...

The invention can further be used for performing an orthologous knock-in in a host organism. Indeed, the methods and compositions according to the present invention can be used to knock-out (inhibit) the native gene of the host organism and an analogous gene from another species, preferably human, can be introduced. This allows to study the analogous gene without the interference of the native gene. Preferably, the host organism is, but is not limited thereto, a mouse, a fish, a nematode.

### LEGEND TO THE FIGURES

Figure 1: Minimal consensus promoter sequence of T7, T3, K11, and SP6 RNAP and corresponding transcripts. Synthetic oligonucleotide templates only need to be double-stranded in the -17 to +1 region of the promoter, and the coding region can be all single-stranded as shown. The +1 base (in bold) is the first base incorporated into RNA during transcription. The underline indicates the minimum sequence required for efficient transcription, bases in red are not strictly required (N: nucleotide, cN: complementary nucleotide).

Figure 2: Structure of antisense oligodeoxynucleotide bond to an example of mRNA target. The antisense oligodeoxynucleotide binds its cognate target via Watson-Crick base pairing.

Figure 3: Structure of ribozyme bond to an example of mRNA target. The ribozyme binds its cognate target via Watson-Crick base pairing. In the target strand, cleavage takes place 3' to the unpaired H (H is an adenine, cytosine or uracile).

Figure 4: Structure of siRNA bond to an example of mRNA target. The siRNA binds its cognate target via Watson-Crick base pairing.

Figure 5: Structure of shRNA which is processed by the cells into siRNA, then binds to an example of mRNA target.

Figure 6 describes the molecules encoding for an antisense RNA molecule (asRNA). Sequences in bold indicate the T7 RNAP promoter sequence. Sequences in grey indicate the extended part T7 RNAP promoter sequence. Sequences in italics indicate the part of the antisense sequence that is optional. ODN refers to "oligonucleotides". The term "T7RNAPp", corresponding to the indicated sequence, will be used in the Figures 7-9.

Figure 7 describes the molecules encoding for a ribozyme. Sequences in bold indicate the T7 RNAP promoter sequence. T7RNAPp indicate the T7 RNAP promoter sequence. Sequences in italics indicate the part of the antisense sequence that is optional. ODN refers to "oligonucleotides". Nucleotides in boxes indicate the catalytic site of ribozyme and corresponding mRNA sequence.

Figure 8 describes the molecules encoding for a siRNA. Sequences in bold indicate the T7 RNAP promoter sequence. T7RNAPp indicate the T7 RNAP promoter sequence. Sequences in italics indicate the part of the antisense sequence that is optional. ODN refers to "oligonucleotides".

Figure 9 describes the molecules encoding for a shRNA. Sequences in bold indicate the T7 RNAP promoter sequence. T7RNAPp indicate the T7 RNAP promoter sequence.

Sequences in italics indicate the part of the antisense sequence that is optional. ODN refers to "oligonucleotides".

Figure 10 describes the molecules encoding for an inhibiting oligonucleotide targeting E.coli beta-galactosidase. The sequences in bold in shRNA corresponds to the loop of shRNA. The underlined sequence in ribozyme corresponds to the catalytic loop of the ribozyme-like oligonucleotides. The bold and italic sequence in ribozyme corresponds to the mRNA site of cleavage of the ribozymes, and corresponding oligonucleotide sequence.

Figure 11 describes the molecules encoding for an inhibiting oligonucleotide targeting the mouse insulin receptor. The sequences in bold in shRNA corresponds to the loop of shRNA. The underlined sequence in ribozyme corresponds to the catalytic loop of the ribozyme-like oligonucleotides. The bold and italic sequence in ribozyme corresponds to the mRNA site of cleavage of the ribozymes, and corresponding oligonucleotide sequence.

### DETAILED DESCRIPTION OF THE INVENTION

The present invention now discloses and provides novel compositions and methods that improve existing oligonucleotide approaches to inhibit gene expression, including antisense oligonucleotides, ribozymes, shRNA and siRNA. As indicated above, the invention involves a cell or a non-human organism expressing a single-subunit RNA polymerase, i.e. the T7 RNA polymerase (T7 RNAP); and molecules comprising a sequence encoding an expression-inhibiting oligonucleotide specific for a targeted gene, operably linked to elements allowing transcription of said sequence by the single-subunit RNA polymerase in the cell or the organism. The introduction of said molecules in said cell or non-human organism results in the transcription of the sequence and the production of an expression-inhibiting RNA oligonucleotide specific for the targeted gene, more particularly a siRNA, shRNA, antisense RNA or ribozymes, depending on the design of oligonucleotides. Therefore, the cells or the non-human organisms present high cellular levels of expression-inhibiting oligonucleotides, more particularly siRNA, shRNA, antisense RNA or ribozymes, resulting in efficient, selective target gene expression inhibition. Accordingly, the main issues of prior art oligonucleotide-mediated gene inhibition methods, i.e. the instability of RNA oligonucleotides and their poor cellular biodisposability, are avoided by the present method, which has the potency to inhibit gene expression in various eukaryotic species at low cost and with nearly immediate results.

The present invention can be used for identifying/studying the function of any targeted gene, for validating a therapeutic target and/or for optimizing expression-inhibiting oligonucleotide such as siRNA, shRNA, antisense RNA and ribozymes.

By "inhibit the expression of a gene" is intended that the production of the gene product (e.g., a protein) is decreased or stopped, typically decreased by at least 5, 10, 20, 50, 75, or 90 % as compared to a control expression. By "inhibit the expression of a gene" is also intended that the production of the gene product (e.g., a protein) is statistically significantly (p<0.05) decreased as compared to a control expression. By "inhibit the expression of a gene" is further intended that the production of the gene product (e.g., a protein) is sufficiently decreased to observe a change in phenotype. This inhibition of the target gene can occur at the transcriptional level and/or to post-transcriptional level (e.g. by degradation of the targeted mRNA) and/or at the translational level.

The present invention has several advantages which lead to an enhanced gene expression inhibition:
- This invention uses low-cost chemically synthesized molecules. These molecules can be either unmodified (phosphodiester) or modified nucleic acid molecules. Such molecules are relatively stable in biological media, therefore allowing their utilization in living organisms.
- Due to the high enzymatic activity of the single-subunit RNA polymerase, more particularly T7 RNAP, the number of cellular copies of inhibiting-oligonucleotides (e.g., antisense RNA, siRNA, shRNA or ribozyme) produced by the cell or organism is high and compensates the poor uptake of oligonucleotides.
- As a result of their synthesis by single-subunit RNA polymerase, more particularly T7 RNAP, the active RNA molecules produced by the cells (e.g., antisense RNA, siRNA, shRNA or ribozyme) accumulate in the cytoplasm and are thus present in the appropriate cell compartment to mediate gene expression inhibition.
- The method results in the production of RNA molecules having various designs, which have a maximum affinity towards the complementary RNA strand and have the potency to form stable complex RNA:RNA duplexes.

### CELLS OR NON-HUMAN ORGANISMS EXPRESSING A SINGLE-UNIT RNA POLYMERASE

### Single subunit RNA polymerases

Single-subunit DNA-dependent RNA polymerases ("RNAP") are among the simplest RNA polymerases known in the art, as no accessory proteins are necessary for specific initiation, elongation, or termination of transcription. The family consists of several enzymes named for the bacteriophages from which they were cloned (T7 RNAP, T3 RNAP, SP6 RNAP, K11 RNAP) (Kochetkov, Rusakova et al. 1998; Tunitskaya and Kochetkov 2002) and the mitochondrial or chloroplast RNAP produced by eukaryotic cells (Tiranti, Savoia et al. 1997).

Therefore, the single-subunit RNA polymerase according to the present invention is a bacteriophage single-subunit RNA polymerase or a mitochondrial RNA polymerase or a chloroplast RNA polymerase, preferably a bacteriophage single-subunit RNA polymerase. In a preferred embodiment, the single-subunit RNA polymerase according to the present invention is selected from the group consisting of T7 RNAP, T3 RNAP, SP6 RNAP, and K11 RNAP. In a most preferred embodiment, the single-subunit RNA polymerase according to the present invention is bacteriophage T7 RNA polymerase (T7 RNAP). In another most preferred embodiment, the single-subunit RNA polymerase according to the present invention is bacteriophage T3 RNA polymerase (T3 RNAP).

Enzymes of this family share strong nucleotide and protein sequence homologies. In addition, they have several characteristics in common: 1 °) As other DNA-dependant RNAP, these enzymes transcribe double-strand DNA into single-strand RNA; 2°) In contrast to eukaryotic and other prokaryotic RNA polymerases, enzymes of this family are single-unit (Kochetkov, Rusakova et al. 1998; Tunitskaya and Kochetkov 2002); 3°) Bacteriophage RNAPs have strong enzymatic activities; 4°) Transcription by bacteriophage RNAPs depends on specific consensus promoter sequences that account for their high specificity (Oakley and Coleman 1977).

From the family of single-subunit DNA-dependant RNA polymerase, T7 RNAP is preferably selected for use in the present invention as this enzyme is well-known and widely used (Kochetkov, Rusakova et al. 1998; Tunitskaya and Kochetkov 2002). Alternatively, other bacteriophage polymerases such as T3 RNAP, K11 RNAP or SP6 RNAP could also be used for the same purpose.

T7 RNAP (EC 2.7.7.6) transcribes late genes of the T7 bacteriophage (Summers and Siegel 1970). The enzyme was first isolated from bacteriophage T7-infected *Escherichia coli* cells in 1970 (Chamberlin, McGrath et al. 1970), and the corresponding gene was cloned in 1984 (Davanloo, Rosenberg et al. 1984). The polypeptide chain of the enzyme consists of 883 amino-acid residues, with a molecular weight of 98.8 kDa (Chamberlin, McGrath et al. 1970).

T7 RNAP catalyzes the synthesis of RNA complementary in sequence to the template DNA in the 5' → 3' direction. At 37°C, about 250 nucleotides are synthesized per second by the enzyme (Golomb and Chamberlin 1974). Maximal enzymatic activity is retrieved at 37°C with a pH of 8.0-9.0, in presence of Mg²⁺ (Kochetkov, Rusakova et al. 1998; Tunitskaya and Kochetkov 2002). T7 RNAP transcripts produced in vivo in the eukaryotic cells are not capped (Dower and Rosbash 2002). However, T7-RNAP transcripts can be spliced, albeit with reduced efficiency (Dower and Rosbash 2002), and can be polyadenylated if they contain a polyadenylation signal (Dower and Rosbash 2002).

### Cells or non-human organisms expressing a single subunit RNA polymerase

The present invention concerns a cell or a non-human organism expressing a single-subunit RNA polymerase as defined above and/or uses thereof. Preferably, said cell is a recombinant cell. By "recombinant cell" is intended herein a cell comprising a heterologous sequence encoding a single-subunit RNA polymerase. By "recombinant cell" is also intended herein a cell comprising a sequence encoding an exogenous RNA polymerase engineered or modified such that the RNA polymerase is expressed in the cytoplasm and/or in the nucleus. In such a context, the exogenous RNA polymerase can be a mitochondrial or chloroplast single subunit RNA polymerase, including an endogenous RNA polymerase. Preferably, the expression of the single-subunit RNA polymerase is achieved by stable transfection (for review, see (Liu, Ren et al. 2003; Nicolazzi, Garinot et al. 2003). Preferably said non-human organism is a transgenic non-human organism (Liu, Ren et al. 2003; Nicolazzi, Garinot et al. 2003). By "transgenic non-human organism or mammal" is intended herein an organism or a mammal comprising a heterologous sequence encoding a single-subunit RNA polymerase. By "transgenic non-human organism or mammal" is also intended herein an organism or a mammal comprising a sequence encoding a RNA polymerase (including an endogenous mitochondrial RNAP) engineered or modified such that the RNA polymerase is expressed in the cytoplasm and/or in the nucleus. In a particular embodiment of the invention, the cell or the non-human organism can express two (or more) different single subunit RNA polymerases, for example T7 and T3 RNA polymerases.

In a particular embodiment, said transgenic non-human organism is obtained by random integration, into the genome of (cells of) said organism, of an expression cassette encoding said single-subunit RNA polymerase. Alternatively, said transgenic non-human organism can be obtained upon integration of the sequence coding for said single-subunit RNA polymerase by homologous recombination (knock-in organism). Methods for preparing said recombinant cells (Sambrook, Fritsch et al. 1989; Liu, Ren et al. 2003; Nicolazzi, Garinot et al. 2003) or non-human transgenic organisms (Hofker and Voncken 2002) are well-known by the man skilled in the art. Methods for generating transgenic organisms, particularly animals such as mice or rats, are described, for example, in US 4,736,866, US 4,870,009, WO 90/11354, and US 5,631,153.

In a first embodiment, the RNA polymerase is constitutively expressed in the cell or the non-human organism. Such constitutive expression may be ubiquitous. In order to obtain a ubiquitous expression, the coding sequence for the single-subunit RNA polymerase is operably linked to an ubiquitous promoter. Such ubiquitous promoters can be of viral, cellular, bacterial or recombinant origin such as, without limitations, promoters of HPRT, PGK, α-actin, or tubulin genes, SV40 early promoter, CMV promoter. Alternatively, the constitutive expression may be tissue-specific. In order to obtain tissue specific expression, the coding sequence for the single-subunit RNA polymerase is operably linked to a tissue specific promoter such as, without limitations, promoters of the pyruvate kinase gene, the villin gene, the gene for intestinal fatty acid binding protein, the smooth muscle α-actin gene.

In a second embodiment, the expression of the RNA polymerase is regulated (e.g., inducible). For example, the coding sequence can be operably linked to a 5'-flanking ecdysone-responsive promoter [Yarovoi, 2001 #1295]. Alternatively, the expression can be controlled by a Tet system (Gossen and Bujard 1992; Gossen, Freundlieb et al. 1995) US 5,464,758; US 5,814,618).

Optionally, the single-subunit RNA polymerase is addressed to the cytoplasm or to the nucleus.

The sequence coding for the RNA polymerase may be integrated into the genome of the recombinant cells or of the cells of the transgenic organisms or episomal, (e.g., extrachromosomal).

T7 RNAP has been constitutively or transiently expressed in mammalian cells (Fuerst, Niles et al. 1986; Chen, Tabor et al. 1987; Fuerst, Earl et al. 1987; Fuerst and Moss 1989; Lieber, Kiessling et al. 1989; Elroy-Stein and Moss 1990; Brisson, He et al. 1999; Dieci, Bottarelli et al. 2000; Yarovoi and Pederson 2001), unicellular parasites, i.e. *Trypanosoma brucei* (Wirtz, Hartmann et al. 1994; Chen, Li et al. 1995; Wirtz, Hoek et al. 1998; Nakano, Nakagawa et al. 2003)), *Arabidopsis thaliana* cells (Dieci, Bottarelli et al. 2000), *Saccharomyces cerevisiae* (Benton, Eng et al. 1990; Dieci, Bottarelli et al. 2000; Dower and Rosbash 2002)) and *Escherichia coli* (Koken, Odijk et al. 1993; Dieci, Bottarelli et al. 2000)). T3 RNAP has also been expressed in *Trypanosoma brucei* (Wirtz, Hartmann et al. 1994). Noticeably, no obvious change of phenotype was observed in the modified cells. Stable transfection of T7 RNA polymerase in eukaryotic cells leads to the expression of the enzyme in the cytoplasm of transfected cells (Elroy-Stein and Moss 1990; Gao and Huang 1993). An example of preparation of stable mammalian cell line expressing a bacteriophage RNA polymerase is disclosed in US 5,126,251, US 4,952,496 and WO 03/050240.

Preferably, a nuclear location signal is added to the coding sequence of RNA polymerase, more preferably at the N terminal end of the RNA polymerase, in order to address the RNA polymerase in the nucleus. The nuclear location signal can be the nuclear location signal of SV40 large T antigen. For example, T7 RNAP polymerase can be addressed to the nucleus of eukaryotic cells by substituting a sequence encoding the nuclear location signal of SV40 large T antigen (e.g., a 36-bp synthetic nucleotide sequence) for the N-terminal part of the polymerase gene T7 gene (Dunn, Krippl et al. 1988; Lieber, Kiessling et al. 1989; Yarovoi and Pederson 2001). Noticeably, this signal does not need to be removed from the protein upon entry to the nucleus (Makkerh, Dingwall et al. 1996).

The cells expressing a RNA polymerase can be prokaryotic or eukaryotic. Prokaryotic cells include bacteria and archaebacteria . Preferably, the cell is eukaryotic. Eukaryotic cells can be yeast, protist, fungi, parasite, animal (including mammalian), plant, or insect cell, etc... In a preferred embodiment, the cell is a mammalian cell. In a most preferred embodiment, the cell is a mouse or human cell, preferably a human cell. The cell can be a primary cell or a cell derived from a cell line. The cell can be a stem cell (preferably an embryonic stem cell), a somatic cell, a gamete, a blastomer or an egg (preferably a fertilized egg). The cell can stem from fish, bird, non-human mammals, insect, amphibian, reptile, preferably from medakafish, zebrafish, mice, rat, chicken, xenopus, sheep, cow, or rabbit, more preferably from fish, chicken, rat and mice. The cell can have all stage of differentiation, from totipotent to differenciated cells. Examples of mammalian cells include human (such as HeLa cells), bovine, ovine, porcine, murine (such as embryonic stem cells), rabbit and monkey (such as COS1 cells) cells. The cell may be an embryonic cell, bone marrow stem cell or other progenitor cell. Where the cell is a somatic cell, the cell can be, for example, an epithelial cell, fibroblast, smooth muscle cell, blood cell (including a hematopoietic cell, red blood cell, T-cell, B-cell, etc...), tumor cell, cardiac muscle cell, macrophage, dendritic cell, neuronal cell (e.g., a glial cell or astrocyte, or pathogen-infected cell 'e.g., those infected by bacteria, viruses, virusoids, parasites, or prions). For example, the cells can be, but are not limited to, COS, CHO, 3T3, HeLa, HepG2, Caco-2, MCF-7, HEK 293 and SW480 cell lines. In a most preferred embodiment, the cell is a NIH/3T3 cell or 3T3-L1 Tet-Off or 3T3-L1 Tet-On cells. It should be understood, however, that the invention is broadly applicable and is not intended to be limited to a particular cell type, nor to particular cell species.

For example, recombinant cells can be prepared by transfection of said cells with a vector comprising the sequence encoding RNAP, more particularly an expression cassette for RNAP. Depending upon the expression vector and transfection technique used, only a small fraction of cells may integrate the foreign DNA into their genome. In order to identify and select these integrants, a gene that encodes a selectable marker (e.g., resistance to antibiotics) is generally introduced into cells along with the expression cassette for RNAP. Various selectable markers include those that confer resistance to drugs, such as G418, hygromycin and methotrexate. Nucleic acid encoding a selectable marker are preferably introduced into cells on the same vector as the RNAP expression cassette. Cells stably transfected with the introduced nucleic acid can be identified by drug selection (e.g., cells that have incorporated the selectable marker gene will survive, while the other cells die).

The transgenic organism can be any animal such as a mouse, a rat, a dog, a cat, a monkey, a lifestock animal (such as a pig, a goat, a sheep or a rabbit), a fish, a frog, , a drosophila, a nematode, or any plant such as crop (including tobacco, maize, wheat, tomato, and rice) or other plants such as Arabidopsis thaliana. In a preferred embodiment, the transgenic animal expressing the RNA polymerase is a non-human mammal, preferably a mouse, a rat, a pig or a rabbit, more preferably a mouse.

For example, a non-human transgenic animal can be produced by transfecting embryonic stem cells with a vector comprising the sequence encoding RNAP and optionally screened for random integration event. The resulting cells are injected into embryos at a stage at which they are capable of integrating the transfected cells, for example at the blastocyst stage, and the embryos are then reimplanted in a surrogate mother. The chimeric individuals with colonization by embryonic stem cells of the germ line are obtained at the end of gestation, are mated to obtain transgenic animals.

Alternatively, a non-human transgenic animal can be produced by transfecting fertilized eggs with a sequence encoding RNAP, more particularly a RNAP expression cassette. Eggs are reimplanted in a surrogate mother, and the transgenic individuals obtained at the end of gestation. Otherwise, the eggs are incubated in condition allowing the growth of the embryo and the generation of the transgenic animal.

T7 RNAP has been expressed in some genetically engineered organisms without detectable change of phenotype. This has been achieved by transgenesis in several species including zebrafish (Verri, Argenton et al. 1997), *Drosophila melanogaster* (Fitzgerald and Bender 2001)), and *Nicotiana benthamiana* (Holt and Beachy 1991; Kollar, Dalmay et al. 1993; Magee and Kavanagh 2002). Genetically engineered animals, i.e. zebrafish (Verri, Argenton et al. 1997) and plants (Magee and Kavanagh 2002), have been used to overexpress reporter genes under the control of the T7 RNAP promoter sequence.

### MOLECULE COMPRISING A SEQUENCE ENCODING AN EXPRESSION-INHIBITING OLIGONUCLEOTIDE

According to the present invention, a molecule is generated and used to synthesize in a cell or in vivo the expression-inhibiting oligonucleotide. These molecules thus contain 1) element(s) required for transcription by the RNA polymerase, and 2) a sequence encoding an expression-inhibiting oligonucleotide specific of a targeted gene operably linked to said element(s). More specifically, the element(s) comprise the promoter of said RNA polymerase, preferably a minimal promoter of said polymerase. Termination occurs when T7 RNA polymerase reaches the 5' end of linearized template or when it reaches a T7 sequence terminator (Lyakhov, He et al. 1997). Optionally, the element(s) comprise a terminator downstream of the sequence encoding the expression-inhibiting oligonucleotide.

The molecules may be of various types, such as a plasmid, vector, DNA or RNA fragment, etc... In a preferred embodiment, the molecule is a synthetic linear or circular DNA molecule consisting essentially of an oligodeoxyribonucleotide.

In a preferred embodiment, the molecule comprises a consensus sequence corresponding to the RNA polymerase consensus promoter sequence and a sequence encoding an expression-inhibiting oligonucleotide specific of the targeted gene operably linked to said consensus promoter sequence; and the molecule is double stranded at least for the promoter sequence.

The sequence encoding an expression-inhibiting oligonucleotide depends on the gene to be targeted and the type of expression-inhibiting oligonucleotide to obtain, i.e. siRNA, ribozyme, shRNA or antisense RNA. The molecule can be single stranded or double stranded for the sequence encoding the expression-inhibiting oligonucleotide. If the molecules are double stranded at least for the promoter sequence, they can be prepared by annealing two oligonucleotide templates that are complementary for the consensus promoter sequence (Milligan, Groebe et al. 1987; Milligan and Uhlenbeck 1989). More particular, the molecule is double stranded at least in the -17 to +1 or -23 to +1 bases of the promoter sequence.

### RNA polymerase consensus promoter sequence

The T7, T3, K11 or SP6 RNAP consensus promoter sequences drive specific initiation of RNA synthesis in the presence of the corresponding polymerase. The minimal promoter sequence consists of 18 base pairs numbered -17 to +1, where +1 is a G nucleotide that starts the transcription (Milligan and Uhlenbeck 1989). The promoter serves at the same time as a recognition motif and a transcriptional start site. Two additional sequence are known to enhance the efficiency of the translation by T7 RNAP but are not strictly required: G nucleotide at +2, and six extra-nucleotides upstream the minimal promoter sequence (nucleotides -23 to -18) (Kochetkov, Rusakova et al. 1998; Tunitskaya and Kochetkov 2002).

Promoters of RNA polymerase enzymes have strong homology, but differ at various positions, especially -8 through -12. In addition, the consensus promoter sequences of these enzymes are highly specific. For instance, T7 RNAP has no affinity for the T3 RNAP consensus promoter sequence (Chamberlin, McGrath et al. 1970).

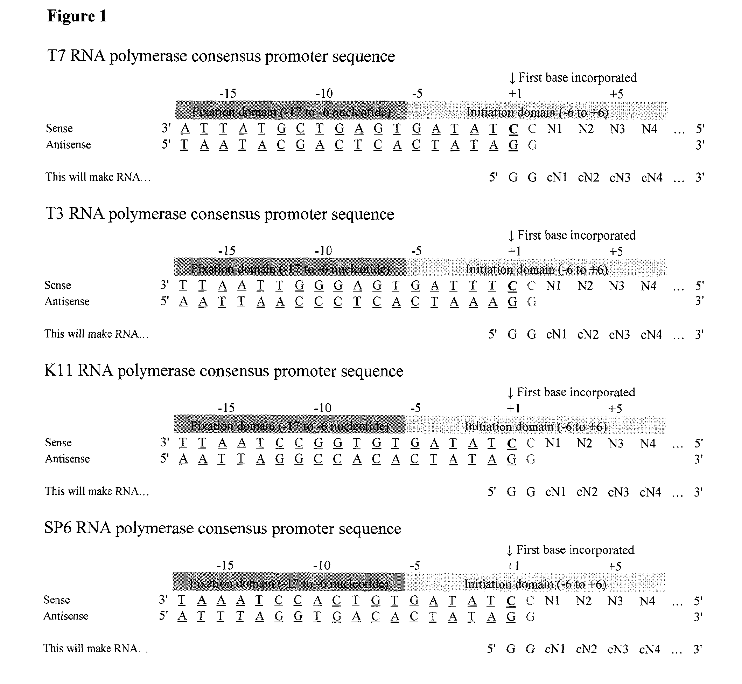

Therefore, the invention concerns more particularly a molecule comprising the minimal promoter sequence from nucleotides -17 through +1 of a single subunit RNA polymerase operably linked to a sequence encoding an expression-inhibiting oligonucleotide. More preferably, the molecule comprises the consensus promoter sequence from nucleotides -17 through +1 of the T7, T3, K11 or SP6 RNAP consensus promoter sequence. Still more preferably, the molecule comprises the T7 RNAP consensus promoter sequence of nucleotides -17 through +1. Examples of sequences are disclosed in Figure 1 (SEQ ID Nos 1-4).

The single-subunit RNA polymerase promoter sequence can optionally comprise 2-10 additional nucleotides upstream of the minimal promoter sequence to enhance the binding of RNA polymerase. Preferably, the promoter sequence comprises 4-8 extra nucleotides upstream of the minimal promoter sequence, more preferably 6 extra nucleotides. More particularly, the molecule comprises nucleotides -23 through +1 or -23 through +2 of the promoter sequence of a single subunit RNA polymerase operably linked to a sequence encoding an expression-inhibiting oligonucleotide. Preferably, said RNA polymerase is selected from the group consisting of T7, T3, K11 and SP6 RNAP. More preferably said RNA polymerase is T7 or T3 RNA polymerase, still more preferably is T7 RNAP.

In addition, the molecule should be double stranded, at least in the promoter sequence. Therefore, two oligonucleotides can be annealed to form a duplex in the promoter sequence. Alternatively, the molecule can be designed to form a hairpin comprising the single-subunit RNA polymerase promoter sequence, and therefore do not require any complementary oligonucleotide to be double stranded at least in the promoter sequence (as described below).

In a particular embodiment of the present invention, the structure of the molecule comprising the sequence encoding the expression-inhibiting oligonucleotide is selected from the group consisting of:
- a hairpin oligonucleotide comprising (or consisting of) a minimal double-stranded RNA polymerase promoter sequence operably linked to a single-stranded oligonucleotide comprising (or consisting of) the sequence encoding the expression-inhibiting oligonucleotide;
- a hairpin oligonucleotide comprising (or consisting of) an extended double-stranded RNA polymerase promoter sequence operably linked to a single-stranded oligonucleotide comprising (or consisting of) the sequence encoding the expression-inhibiting oligonucleotide;
- a hairpin oligonucleotide comprising (or consisting of) a minimal double-stranded RNA polymerase promoter sequence operably linked to a double-stranded oligonucleotide comprising (or consisting of) the sequence encoding the expression-inhibiting oligonucleotide;
- a hairpin oligonucleotide comprising (or consisting of) an extended double-stranded RNA polymerase promoter sequence operably linked to a double-stranded oligonucleotide comprising (or consisting of) the sequence encoding the expression-inhibiting oligonucleotide;
- an oligonucleotide comprising (or consisting of) a minimal double-stranded RNA polymerase promoter sequence operably linked to a single-stranded oligonucleotide comprising (or consisting of) the sequence encoding the expression-inhibiting oligonucleotide;
- an oligonucleotide comprising (or consisting of) an extended double-stranded RNA polymerase promoter sequence operably linked to a single-stranded oligonucleotide comprising (or consisting of) the sequence encoding the expression-inhibiting oligonucleotide;
- an oligonucleotide comprising (or consisting of) a minimal double-stranded RNA polymerase promoter sequence operably linked to a double-stranded oligonucleotide comprising (or consisting of) the sequence encoding the expression-inhibiting oligonucleotide;
- an oligonucleotide comprising (or consisting of) an extended double-stranded RNA polymerase promoter sequence operably linked to a double-stranded oligonucleotide comprising (or consisting of) the sequence encoding the expression-inhibiting oligonucleotide.

Examples of hairpin oligonucleotide comprising a minimal or extended double-stranded T7 RNA polymerase promoter sequence are provided in SEQ ID Nos 5 and 6.

In a less preferred embodiment of the present invention, the oligonucleotide comprising (or consisting of) a minimal or extended double-stranded RNA polymerase promoter sequence operably linked to a double-stranded oligonucleotide comprising (or consisting of) the sequence encoding the expression-inhibiting oligonucleotide is inserted in a vector. Such vector can be a plasmid or a viral vector.

The T7 RNAP system has already been used to increase the cellular expression of plasmids containing a reporter gene under control of the T7 RNAP promoter sequence. This technology has been used in mammalian cells (Fuerst, Niles et al. 1986; Fuerst, Earl et al. 1987; Fuerst and Moss 1989; Elroy-Stein and Moss 1990; Brisson, He et al. 1999), yeast (Benton, Eng et al. 1990), parasites (Wirtz, Hartmann et al. 1994; Wirtz, Hoek et al. 1998), and bacterial cells (Koken, Odijk et al. 1993; Delano, Dombrowski et al. 1999). Similar results were obtained with the T3 RNA polymerase expression system to overexpress reporter genes (Deuschle, Pepperkok et al. 1989). In addition, the T7 RNAP expression system can also be used to synthesize non-coding RNA, i.e. tRNA (Dieci, Bottarelli et al. 2000). However, it has never been suggested to specifically express an inhibiting oligonucleotide according to the present invention.

The cell compartment where this synthesis occurs, i.e. the cytoplasm and/or the nucleus, depends on the addressing of the single-subunit RNA polymerase. Noticeably, the pH of the nucleus (7.5-8.0) and the cytoplasm (7.0-7.5) of eukaryotic cells is compatible with the biological activity of single-subunit RNA polymerase, more particularly T7 RNAP (Seksek and Bolard 1996).

### Expression-inhibiting oligonucleotide

The expression-inhibiting oligonucleotide according to the present invention is any oligonucleotide specific for a targeted gene which inhibits or decreases the expression of the targeted gene. More particularly, the expression-inhibiting oligonucleotides are mostly RNA molecules. The preferred expression-inhibiting oligonucleotides are selected from the group consisting of antisense oligonucleotides, shRNA, ribozymes and siRNA.

Any gene expressed by the cell can be inhibited by the method according to the present invention. Target genes include not only coding genes, but also any type of non-coding genes such as snRNA, microRNAs, ribozymes, tRNA, rRNA and others. In addition, the present technology overcomes the problem of lethality caused by gene expression inhibition during embryogenesis.

Moreover, the method according to the present invention is based on direct utilization of gene sequence information. Therefore, there is no need of complete knowledge of the sequence of the targeted gene. Consequently, this approach is well-suited for the gene predictions, such as cluster of ESTs (expressed sequence tags) resulting from genome sequencing analysis.

In addition, the expression of specific splice variants of a gene can be inhibited by designing oligonucleotides that cover exon-exon junctions and/or exons only present in the splice variants to be targeted.

Depending on the design of oligonucleotides, different RNA molecules can be produced by the cells, including shRNA, siRNA, antisense RNA and ribozymes, thereby providing several advantages:
- The duration of gene inhibition differs according to the type of RNA. For instance, gene expression inhibition by antisense oligonucleotides is likely brief (∼1 day), intermediate for ribozymes (3-4 days) (Johnson and Ball 1999), while gene expression inhibition by siRNA and shRNA is expected to be much longer (∼3 to 7 days). This offers the possibility of choosing the duration of gene expression inhibition.
- In contrast to siRNA-like and shRNA-like approaches, ribozyme-like and antisense RNA-like do not take advantage of cellular protein machinery. Therefore, both ribozyme-like and antisense RNA-like approaches will be non-saturable and can be likely used to inhibit several genes simultaneously.

Depending on the approach selected, the method according to the present invention can be used in various species. For hammerhead ribozyme, gene expression inhibition only depends on intrinsic catalytic activity of the molecules (Lipardi, Wei et al. 2001). Therefore, ribozyme-like approach is supposed to be compatible with any type of cell from any species. For antisense RNA, gene expression inhibition is supposed to depend mainly on ribosome blocking (Kurreck 2003). Therefore, RNA antisense-like approach is thought to be compatible with most species. For siRNA and shRNA, RNA inhibition was shown to exist in various species, including mammalian and non-mammalian vertebrates, plants and lower eukaryotes. siRNA- and shRNA-like approaches are therefore expected to be compatible with all these species.

### Antisense oligonucleotides

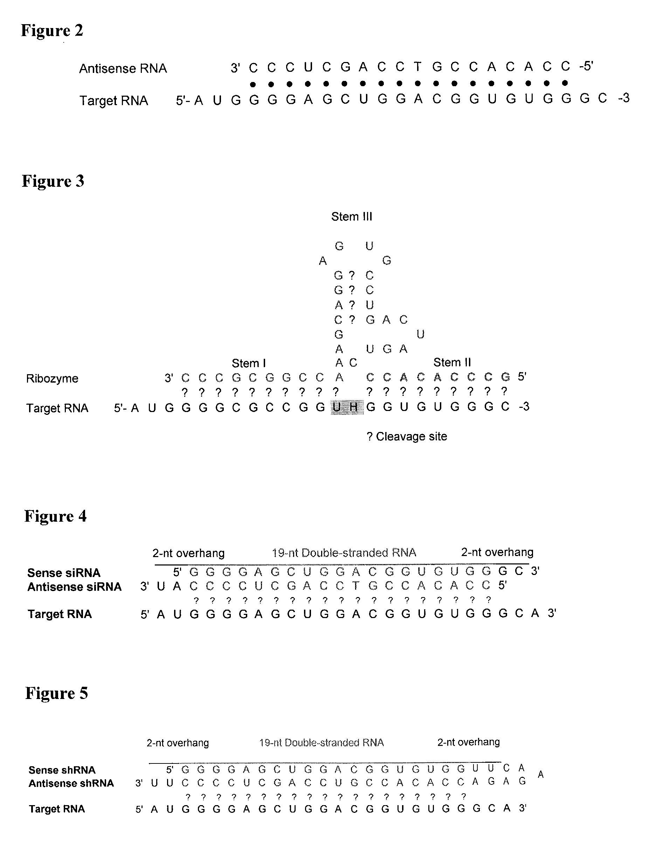

Antisense oligonucleotides are short single-strand molecules that are complementary to the target mRNA and typically have 10-50 mers in length, preferably 15-30 mers in length, more preferably 18-20 mers in length (Gewirtz, Sokol et al. 1998). For example, see Figure 2. Antisense oligonucleotides are preferably designed to target the initiator codons, the transcriptional start site of the targeted gene or the intron-exon junctions (Gewirtz, Sokol et al. 1998).

Antisense oligonucleotides are thought to inhibit gene expression through various mechanisms: 1°) Degradation of the complexes between target RNA/DNA oligonucleotide by RNase H. The latter is a ubiquitous nuclear enzyme required for DNA synthesis, which functions as an endonuclease that recognizes and cleaves the RNA in the duplex. Most types of oligonucleotides, but not all, from complexes with mRNA that direct the cleavage by RNase H (Dagle, Weeks et al. 1991; Opalinska and Gewirtz 2002); 2°) Inhibition of translation by the ribosomal complexes (Shakin and Liebhaber 1986; Opalinska and Gewirtz 2002), 3°) Competition for mRNA splicing when oligonucleotides are designed against intron-exon junctions (Dominski and Kole 1994; Opalinska and Gewirtz 2002).

### Ribozymes

Ribozymes are single stranded RNA molecules retaining catalytic activities. Their structures are based on naturally occurring site-specific, self-cleaving RNA molecules. Five classes of ribozymes have been described based on their unique characters, i.e. the Tetrahymena group I intron, RNase P, the hammerhead ribozyme, the hairpin ribozyme and the hepatitis delta virus ribozyme (Doudna and Cech 2002).

The hammerhead ribozyme at about 40 nucleotides shares similarities with the shape of a hammerhead. For example, see Figure 3. They are the most common and the smallest of the naturally occurring ribozymes. Their normal function is to process multimeric viral RNAs into monomers (Doudna and Cech 2002). Interestingly, hammerhead motif was shown as the most efficient self-cleaving sequence that can be isolated from randomized pools of RNA (Salehi-Ashtiani and Szostak 2001). Hammerhead ribozymes-like oligonucleotides are presently used because of their effectiveness and their short sequence.

The hammerhead ribozyme consists of three short stems: stems I and II are designed to anneal with a specific RNA sequence, whereas stem-loop III contains a catalytic cleavage site. Once bound, hammerhead ribozyme cleaves the phosphodiester backbone of the target RNA by a transesterification reaction. The catalytic moiety of ribozymes recognize specific 5'-UH-3' sites of the mRNA target, where U is a uracile and H is an adenine, cytosine or uracile. The mechanism of cleavage is free of any protein factor, but requires the presence of bivalents metal cations, e.g. Mg²⁺. Once they have cleaved their target, ribozymes are released from their mRNA target and are free to cleave another mRNA molecule (Usman and Blatt 2000).

It has been shown that unmodified and modified (2'-O-methyl and phosphorothioate) synthetic ribozymes can be used to inhibit specifically gene expression in vitro (Usman and Blatt 2000). It has also been shown that inhibition of gene expression can be also achieved by administration of synthetic modified ribozymes to living organisms (Czubayko, Schulte et al. 1996; Lee, Blatt et al. 2000; Abounader, Lal et al. 2002; Aigner, Fischer et al. 2002)

### siRNA

RNA interference (RNAi), quelling or post-transcriptional gene silencing (PTGS) designate a phenomenon by which dsRNA specifically suppresses expression of a target gene at post-translational level. This mechanism, which was originally discovered in nematode worm (*Caenorhabditis elegans*) (Fire, Xu et al. 1998), has now been found in a large number of organisms including fungi (Neurospora crasa (Romano and Macino 1992)), parasites (*Tryponosomia brucei* (Ngo, Tschudi et al. 1998)), *Planaria* (Sanchez Alvarado and Newmark 1999), *Hydra* (Lohmann, Endl et al. 1999), *Drosophila* (Misquitta and Paterson 1999), zebra fish (Wargelius, Ellingsen et al. 1999), plants (Angell and Baulcombe 1997; Ruiz, Voinnet et al. 1998), and mammalians (Elbashir, Harborth et al. 2001; McManus and Sharp 2002).

In normal conditions, RNAi is initiated by double-stranded RNA molecules (dsRNA) of several thousands of base pair length. In vivo, dsRNA introduced into a cell is cleaved into a mixture of short dsRNA molecules called short interfering RNA (siRNA). The enzyme that catalyzes the cleavage, Dicer, is an endo-RNase that contains RNase III domains (Bernstein, Caudy et al. 2001). The siRNAs produced by Dicer are 21-23 bp in length in mammalian cells (Hamilton and Baulcombe 1999; Zamore, Tuschl et al. 2000; Bernstein, Caudy et al. 2001; Elbashir, Harborth et al. 2001; Elbashir, Lendeckel et al. 2001; Grishok, Pasquinelli et al. 2001)

Reduction of gene expression usually occurs within the 24 hours after treatment, and persists for the 5-10 following days (Li, Lin et al. 2003). Gene inhibition by siRNA is an efficient process which is at least tenfold more potent as silencing trigger than sense or antisense RNAs alone (Fire, Xu et al. 1998). In some species as worms and plants but not mammals (Hannon 2002), directed silencing reaction can spread throughout the organism. This phenomenon, called transitive RNAi, suggests the existence of a cell-to-cell transmission of RNAi in plants and worms (Fire, Xu et al. 1998; Lipardi, Wei et al. 2001; Hannon 2002).

In contrast to long dsRNA molecules (>38 bp), siRNA do not activate the non-specific apoptotic pathway and can therefore be used to inhibit specifically gene expression (Fire, Xu et al. 1998). siRNA are usually designed against a region 50-100 nucleotides downstream the translation initiator codon, whereas 5'UTR (untranslated region) and 3'UTR are usually avoided. For example, see Figure 4.

Short hairpin RNA molecule (shRNA) that are processed by the cells into a siRNA can be also used to degrade the target mRNA and suppress expression (Brummelkamp, Bernards et al. 2002; Paddison, Caudy et al. 2002). For example, see Figure 5.

siRNA and shRNA can be produced by several means including T7 RNA polymerase promoters (Donze and Picard 2002; Paddison, Caudy et al. 2002). In addition, siRNA and shRNA can be produced in living organisms by insertion of lentiviral vectors (Abbas-Terki, Blanco-Bose et al. 2002; An, Xie et al. 2003; Scherr, Battmer et al. 2003). Interestingly, gene inhibition can be obtained in living nematodes by feeding them with bacteria engineered to express long-range double-strand RNA (Timmons and Fire 1998).

Gene inhibition by siRNA appears to be more efficient when genes expressed in liver are targeted (Lewis, Hagstrom et al. 2002; Klein, Bock et al. 2003; McCaffrey, Meuse et al. 2003; McCaffrey, Nakai et al. 2003; Song, Lee et al. 2003).

### Sequence encoding the expression-inhibiting oligonucleotide

The sequence encoding the expression-inhibiting oligonucleotide typically comprises a sequence encoding an oligonucleotide which specifically hybridizes to the targeted mRNA. Perfect complementarity to targeted mRNA is preferred, to ensure high specificity. However, certain mismatch may be tolerated.

Preferably, the last base of the target is a C because bacteriophage RNA polymerase often puts a G at the start of all transcripts. More preferably, the two last bases of the target are C because bacteriophage RNA polymerase often puts two G at the start of all transcripts.

### Antisense - like oligonucleotides

The sequence encoding an antisense is a 10-mers to a 50-mers long and designed against the coding sequence of the targeted mRNA. In preferred embodiment, the sequence encoding an antisense is about a 20-mers long designed against the coding sequence of the targeted mRNA.

Other criteria used for their selection are the following:
- Lack of allelic polymorphism in the targeted mRNA sequence (except if the goal is to inhibit specifically the expression of an allelic variant);
- Lack of significant gene homology in the species in which the experiment is carried out;
- GC content of the target sequence: 40%-60%;
- Antisense oligonucleotide binding energy = -10 kcal/mol;
- Lack of GGGG and AAAA repeat in the targeted sequence;
- Lack of AUCUGUU T7 RNAP termination signal; and/or
- No stable homoduplex between the 3' ends of RNA products.

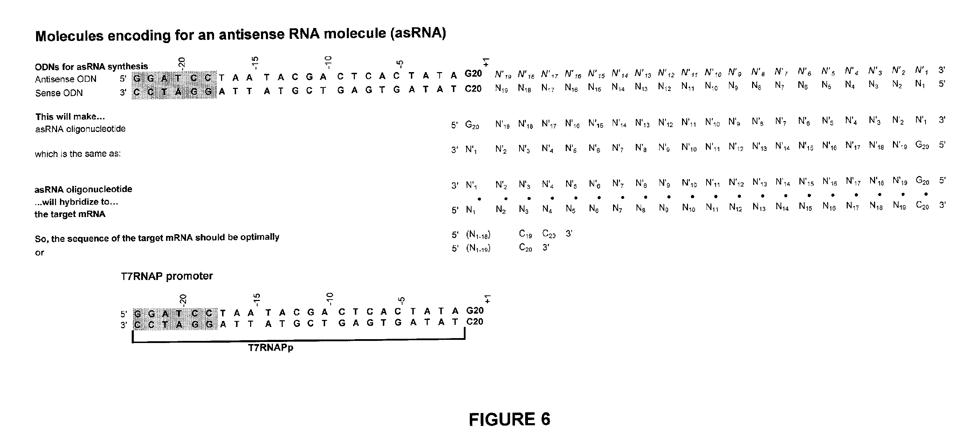

To be transcribed by a bacteriophage RNA polymerase, more particularly T7 RNAP, targeted mRNA sequence should be preferentially 5'-N₁₋₁₈ G₁₉G₂₀ 3', or if lacking 5'N₁₋₁₉ G₂₀ 3'.
Details of oligonucleotides sequence for the T7 RNAP are given Figure 6.

### Ribozyme - like oligonucleotides

The sequence encoding a ribozyme is designed to anneal a sequence of 10-mers to 50-mers long of the targeted mRNA, preferably a sequence of 20-mers long of the targeted mRNA. The target mRNA must contain a cleavage NUH triplet, preferentially GUC.

Other criteria used for their selection are the following:
- Lack of allelic polymorphism in the targeted mRNA sequence (except if the goal is to inhibit specifically the expression of an allelic variant);
- Lack of significant gene homology in the species in which the experiment is carried out;
- GC content of the target sequence: 40%-60% ;
- Antisense oligonucleotide binding energy = -10 kcal/mol;
- Lack of AAAA, CCCC, GGGG, or UUUU repeat in the target sequence;
- Lack of AUCUGUU T7 RNAP termination signal; and/or
- No stable homoduplex between the 3' ends of RNA products.

To be transcribed by a bacteriophage RNA polymerase, more particularly T7 RNAP, target mRNA sequence should be preferentially 5'-(N₁₋₈)*G*_{*9*}U₁₀H₁₁(N₁₂₋₁₈)C₁₉C₂₀-3' (G₁₉ nucleotide is likely to enhance the transcription by RNA polymerase), or if lacking 5'-(N₁₋₈)*G*_{*9*}U₁₀H₁₁(N₁₂₋₁₉)C₂₀-3', or if lacking other sequence containing following previous rules and containing a GAC cleavage triplet or another one.

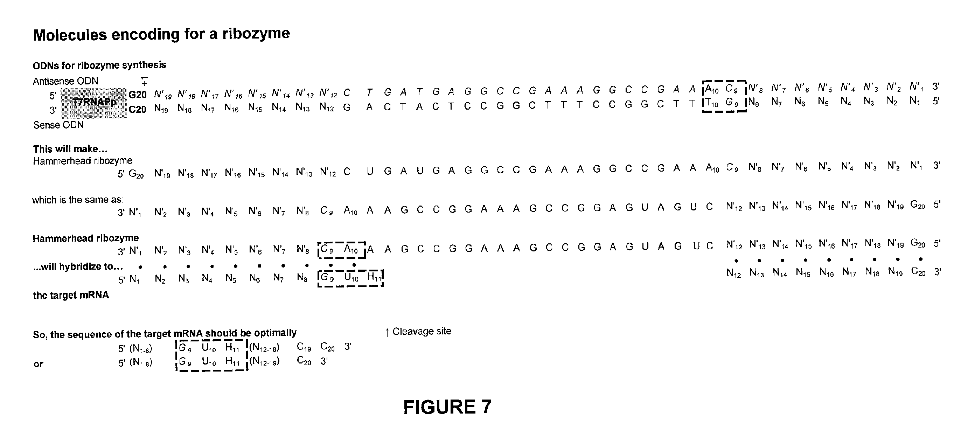

Details of oligonucleotides sequence for the T7 RNAP are given Figure 7.

### siRNA - like oligonucleotides

The sequence encoding a siRNA is 10-50 nucleotides in lenght, with a duplex sequence and 3' uridine overhangs. Preferably, the sequence encoding a siRNA is 21 or 22 nucleotides long, with a 19 or 20 nucleotides duplex sequence and 3' uridine overhangs (Elbashir, Lendeckel et al. 2001). The siRNA are designed against the coding sequence of the targeted gene, while 5'UTR (untranslated region) and 3'UTR are avoided. The secondary structure of the mRNA has important effects on the potency of siRNAs (Lee, Dohjima et al. 2002; Bohula, Salisbury et al. 2003; Vickers, Koo et al. 2003).

Other rules are the following:
- Lack of allelic polymorphism in the targeted mRNA sequence (except if the goal is to inhibit specifically the expression of a given allelic variant);
- Lack of significant homology with other genes of the species in which the experiment is carried out;
- GC content of the target sequence (excluding the 3' nucleotides which are not involved in the base pairing between the target mRNA and the antisense strand of siRNA): 30%-60%;
- Antisense siRNA binding energy = -15 kcal/mol ;
- Lack of GC repeat =7 base-pairs;
- Lack of AAAA, CCCC, GGGG, or UUUU repeat in the target sequence;
- Lack of AUCUGUU T7 RNAP termination signal; and/or
- No stable homoduplex between the 3' ends of RNA products.

For siRNA, two duplex oligonucleotides are required. Two molecules are designed to synthesize each strand of the siRNA molecule.

To be transcribed by a bacteriophage RNA polymerase, more particularly T7 RNAP, target mRNA sequence should be preferentially 5'-X₁X₂G₃G₄(N₅₋₁₉)C₂₀C₂₁X₂₂X₂₃-3', or if lacking 5'-X₁X₂G₃(N₄₋₂₀)C₂₁X₂₂X₂₃-3'.

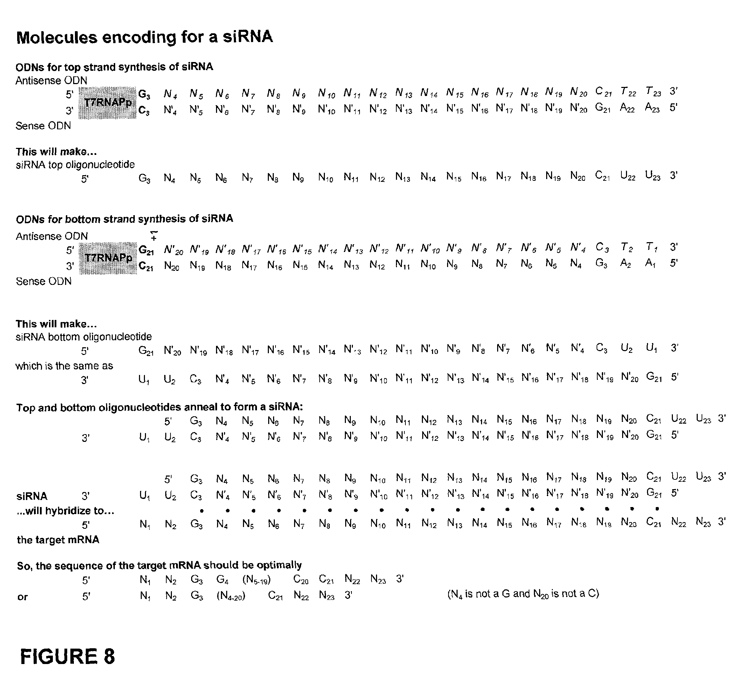

Details of oligonucleotides sequence for the T7 RNAP are given Figure 8.

### shRNA - like oligonucleotides

The sequence encoding a shRNA is designed with the same rules than for a sequence encoding a siRNA. The additional characteristic is several nucleotides (4-10, preferably 6 nucleotides) forming a loop between the two strands of the siRNA.

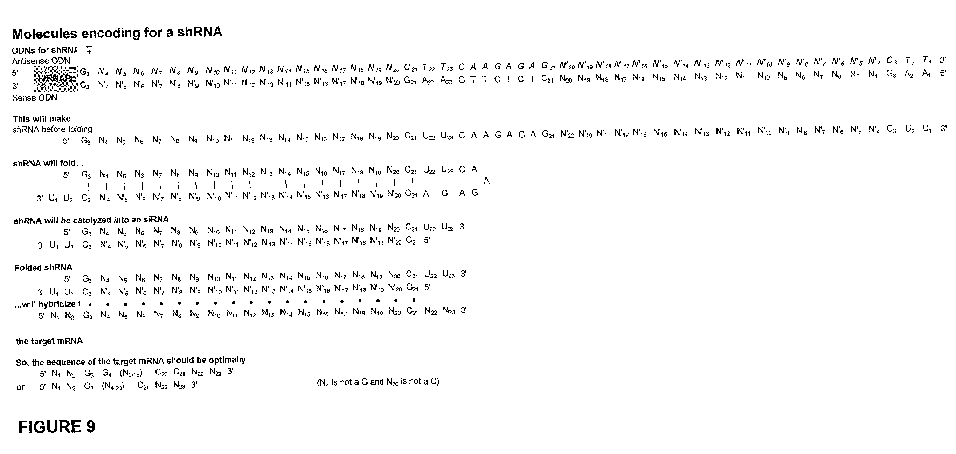

Details of oligonucleotides sequence for the T7 RNAP are given Figure 9.

Different designs of molecules have been used in vitro in order to produce short transcripts such as ribozymes (Hoffinan and Johnson 1994), siRNA (Donze and Picard 2002; Yu, DeRuiter et al. 2002) and shRNA (Yu, DeRuiter et al. 2002; Katoh, Susa et al. 2003). The treatment of eukaryotic cells with T7 RNAP-synthesized shRNA (Yu, DeRuiter et al. 2002; Katoh, Susa et al. 2003) and siRNA (Donze and Picard 2002; Yu, DeRuiter et al. 2002) result in an inhibition of expression of the targeted gene.

### Chemical synthesis and purification

The molecule comprising a sequence encoding an expression-inhibiting oligonucleotide is essentially an oligonucleotide, preferably a oligodeoxyribonucleotide (ADN). Said oligonucleotide can be unmodified, partly modified (more particularly at the ends) or fully modified. Furthermore, the molecule can be labeled. For example, said molecule can be linked to a fluorescent label or to a biotin.

Interestingly, several types of modified oligonucleotides can be transcribed by the T7 RNA polymerase, including phosphorothioate oligonucleotides (Burgin and Pace 1990; Alt, Renz et al. 1997), and circular double-strand 2'-deoxyribo-oligonucleotides (Azhayeva, Azhayev et al. 1997).

The molecules can be routinely synthesized by the β-cyanoethyl phosphoramidite method. Unmodified oligonucleotides, named phosphodiester oligonucleotides, are rapidly degraded by endo- and exo-nucleases, e.g. the highly active 3'-exonuclease. Yet, several modifications of the nucleotide can be distinguished including analogs with modified sugars (especially at the 2' position of the ribose) or phosphate backbones, and others. Only the most studied types of oligonucleotides will be described thereafter:
- Phosphorothioate oligonucleotides result of a change of the non-bridging oxygen atom of the phosphate group into a sulfur atom. This change improves significantly the resistance of oligonucleotides to nucleases the half-life of phosphorothioate oligonucleotide in human serum being ∼9-10 hours compared to ∼1 hour for non-modified oligonucleotides (Kurreck, Wyszko et al. 2002). However, these oligonucleotideS are lipophobic polar molecules resulting in a poor intake by the cells, which is mainly achieved by an endocytosis mechanism. Because the sulfur group of phosphorothioate oligonucleotides are toxic in living organisms, oligonucleotides containing only a few phosphorothioate moieties at their extremities, i.e. MBOs (mixed backbone oligonucleotides that), have been tested and show reduced toxicity (Gewirtz, Sokol et al. 1998).
- Methyl-phosphonate oligonucleotides also result of a change of the non-bridging oxygen atom of the phosphate group into a CH₃ group. This change improves significantly the resistance of oligonucleotides to nucleases. Interestingly, these oligonucleotides are lipophilic due to their neutral charge and are therefore thought to diffuse passively in the cytoplasm of cells (Gewirtz, Sokol et al. 1998).
- N3'→P5' phosphoramidate oligonucleotides result of a change of the 3'-hydroxyl group of the 2'-deoxyribose ring by a 3'-amino group. These oligonucleotides are highly nuclease-resistant and form extremely stable complex with single-stranded RNA (Gewirtz, Sokol et al. 1998).
- Peptide nucleic acid (PNA) result of a change of the 3'-oxygen atom of the phosphate group into the neutral polyamine radical. PNAs are highly nuclease-resistant and form very stable complex with single-stranded RNA (Gewirtz, Sokol et al. 1998). Improved intracellular could be ontained by coupling PNAs to negatively charged oligomers, lipids or certains peptides that are efficiently internalized by cells (Kurreck, Wyszko et al. 2002). PNAs appear to be nontoxic, as they are uncharged molecules with low affinity for proteins that normally bind to nucleic acids.
- 2'-O-methyl and 2'-O-methoxy-ethyl RNAs result of a modification of the 2'-oxygen of the ribose group. Both types of oligonucleotides are highly nuclease-resistant (Gewirtz, Sokol et al. 1998).
- Locked nucleic acid oligonucleotides (LNA) contains a methylene bridge that connects the 2'-oxygen of the ribose with the 4'-carbon of a ribonucleotide. LNAs have high stability in biological medium, increase melting temperature (Kurreck, Wyszko et al. 2002).
- Conjugated oligonucleotides result of the conjugation of oligonucleotides with cell-penetrating peptides typically of 15-50 amino-acids. Various peptide have been tested, the most widely recognized being the Antennapedia (Ant) and HIV-1 TAR delivery peptides (Tung, Wang et al. 1995; Chaloin, Vidal et al. 1998; Astriab-Fisher, Sergueev et al. 2002; Fisher, Ye et al. 2002).

Some additional modifications can be used in conjunction to the previous chemical changes to enhance the stability of oligonucleotides:
- Inversion of the thymidine at their 3' end (Kurreck 2003)
- Circularization of DNA molecules resulting in nuclease-resistant oligonucleotides because they lack of 5' or 3' extremities. If they are self complementary, circular oligonucleotides can self-anneal.

Following the synthesis phase, purification is usually required to remove the salts used for oligonucleotides synthesis. Oligonucleotides purification can be achieved by various means, including desalting, cartridge, reverse phase HPLC (high performance liquid chromatography) and PAGE (polyacrylamide gel electrophoresis). PAGE and HPLC, which provide the highest degree of purity, is usually required for oligonucleotides greater than 40 bases such as those presently used.

### Cellular uptake and trafficking of molecules

Naked phosphorothioate and phosphodiester oligodeoxynucleotides are poorly taken up by cells (Stein, Tonkinson et al. 1993). Their charge state makes it very difficult for them to diffuse passively across the membranes of the cells. Thus, their uptake is thought to be an active energy-dependent process that depends on receptor-like mechanisms and fluid-phase endocytosis processes. Cellular uptake can be significantly increased by the utilization of cationic lipids with lamellar structure, i.e. liposomes, or merely coating the DNA (Kurreck 2003). Following membrane uptake, phosphorothioate and phosphodiester oligonucleotides progress through the lysosomal and endosomal compartments. Oligonucleotides that escape from the lysosomal/endosomal compartments accumulate in the nuclei of eukaryotic cells (Clarenc, Lebleu et al. 1993; Gao, Storm et al. 1993; Beltinger, Saragovi et al. 1995; Aoki, Morishita et al. 1997; Oehlke, Birth et al. 2002). This limited ability to escape these compartments and reach the intracellular sites of action likely account for the poor efficiency of oligonucleotides in inhibiting gene expression.

In contrast, methylphosphonate oligodeoxynucleotides, which are uncharged molecules, are supposed to enter the cells via passive diffusion and to be distributed to the cytoplasm of the cells (Miller 1991; Tari, Andreeff et al. 1996).

### EXAMPLES

### Materials and Methods

### Oligonucleotides

### Chemical synthesis

The oligonucleotides are synthesized by β-cyanoethyl phosphoramidite method and purified by PAGE or HPLC.

A first batch of tested oligonucleotide includes phosphodiester oligonucleotides and phosphorothioate oligonucleotides.

A second batch of tested oligonucleotide includes methylphosphonate, 2'-O-methyl oligonucleotides, PNAs, 2'-O-(2-methoxy)ethyl phosphorothioate oligonucleotides (MOE) with five nucleotides on both 3'- and 5-termini containing a 2'-O-(2-methoxy)ethyl group, oligonucleotides conjugated to a peptide sequence, and LNAs.

In addition, four different types of protection are compared, i.e. none, 3' inverted thymidine, 5-methyl cytosine at 3' extremities and circularization of oligonucleotides.

### Design of the oligonucleotides to be transcribed by the T7 RNA polymerase

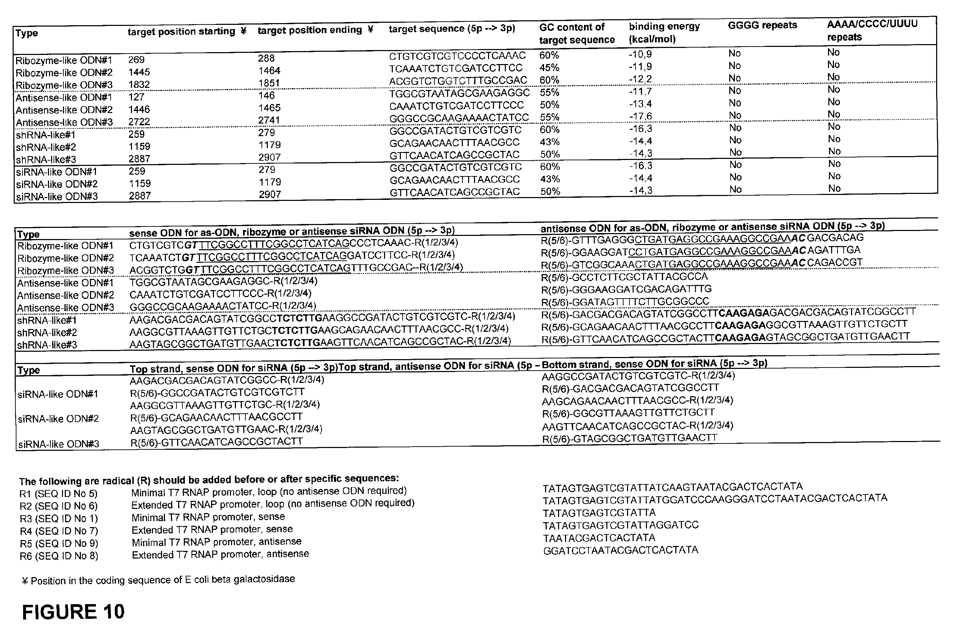

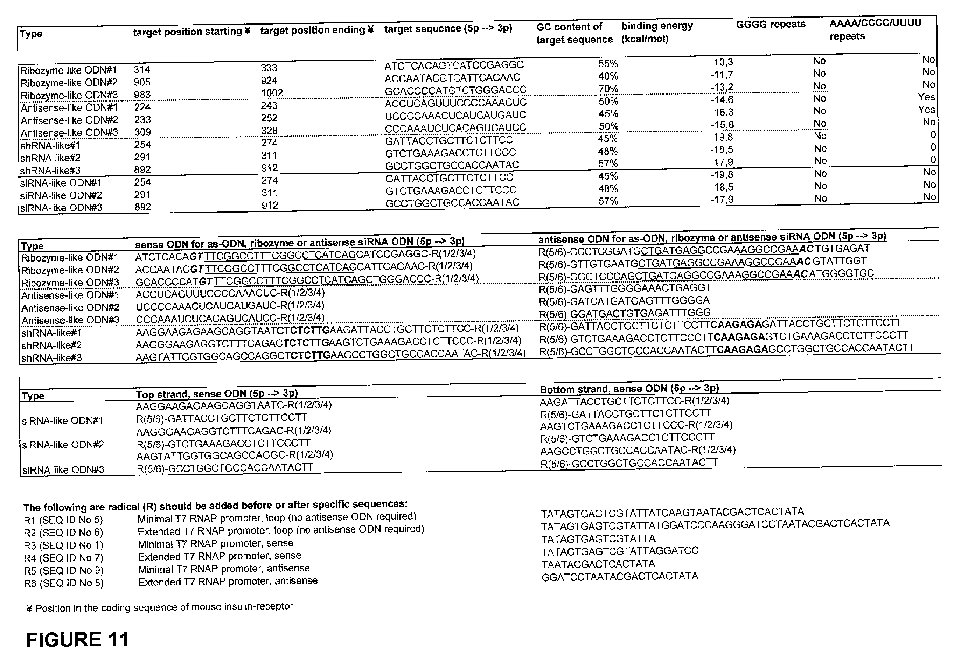

Eight designs of oligonucleotides are tested (see Figures 10 and 11):
- Single-stranded targeting sequence, with a minimal double-stranded promoter sequence. A single oligonucleotide that forms a loop linking T7 RNAP promoter sequences is used.
- Single-stranded targeting sequence, with an extended double-stranded promoter sequence. A single oligonucleotide that forms a loop linking T7 RNAP promoter sequences is used.
- Double-stranded entire oligonucleotide sequence, with a minimal promoter sequence. A single oligonucleotide that forms a loop linking T7 RNAP promoter sequences is used.
- Double-stranded entire sequence, with an extended promoter sequence. A single oligonucleotide that forms a loop linking T7 RNAP promoter sequences is used.
- Single-stranded targeting sequence, with a minimal double-stranded promoter sequence. Two complementary oligonucleotides are annealed.
- Single-stranded targeting sequence, with an extended double-stranded promoter sequence. Two complementary oligonucleotides are annealed.
- Double-stranded entire sequence, with a minimal promoter sequence. Two complementary oligonucleotides are annealed.
- Double-stranded entire sequence, with an extended promoter sequence. Two complementary oligonucleotides are annealed.

Sequences of the selected molecule encoding an antisense, a ribozyme, a siRNA or a shRNA directed against β-galactosidase are given in Figure 10. Sequences of the selected molecule encoding an antisense, a ribozyme, a siRNA or a shRNA directed against mouse insulin receptor are given in Figure 11.

In order to anneal oligonucleotides, the same amounts of the antisense and sense oligonucleotides are mixed, then heated at 95°C for 3 minutes and allowed to cool slowly to room temperature.

### Production of transfected cells expressing the T7 RNA polymerase

### T7 RNA polymerase gene

Two inserts are produced by PCR using as a template the vector pAR1151 (ATCC clone #39561; GenBank sequence J02518). This plasmid contains the wild-type bacteriophage T7 RNA polymerase gene (nucleotide position within the vector: 3145 to 5841).

Two couples of primers will be used in order to generate these PCR products:
- PCR product #1 (total length: 2668 nucleotides): 5'- *PstI* cloning site; Kozac sequence with ATG codon; in frame sequence of the T7 RNA polymerase gene with stop codon; and *SalI* cloning site-3'.
- PCR product #2 (total length: 2689 nucleotides): 5'- *PstI* cloning site; Kozac sequence with ATG codon; in frame NLS (nuclear location signal) sequence of SV-40, in frame wild-type sequence of the T7 RNA polymerase gene with stop codon; and *SalI* cloning site-3'.

Amplification of the template is carried out with a Taq-polymerase having proof-reading activity, such as the Vent DNA polymerase (New England BioLabs).

Both amplicons are purified, and then subjected to restriction digestion by *SalI* and *PstI*.

### Expression vector and Tet-system

To monitor the expression of a reporter gene, i.e. β-galactosidase, cells having a functional Tet system and expressing stably T7 RNA polymerase are produced. This system is setting up by creating a double stable Tet cell line which contains both regulatory and response plasmid.
- The regulatory plasmid is expressed by the 3T3-L1 Tet-Off cells (Clontech)
- The response plasmid that is selected is pBG-I (Clontech). This plasmid contains a bidirectional promoter that is responsive to the tTA regulatory proteins in the Tet-Off system and has two minimal CMV promoters (P_{minCMV}). One of these promoters controls the expression of beta-galactosidase that is used as a reporter gene. The second promoter regulates the expression of the gene of interest, which is presently the bacteriophage T7 RNA polymerase.

Other important patterns of the Tet system and pBI-G vector are the following:
- Maximal expression levels in Tet systems are very high and compare favorably with the maximal levels obtainable from strong, constitutive mammalian promoters such as CMV;
- Background expression of response gene in the absence of induction is extremely low; and,
- No pleiotropic effects of the Tet-system, presumably because the regulatory DNA sequences introduced in the cells are nonexistent in eukaryotic genomes.

The main steps of the construction of the pBI-G/T7RNAP vector are the following:
- Digestion of the pBI-G vector by *SalI* and *PstI* restriction enzymes, treatment with phosphatase, and purification;
- Blunt-end oriented ligation of each insert into the pBI-G vector;
- Propagation of the vector in DH5a *E*. *coli* strain, and selection with ampicillin;
- Identification of the desired recombinant plasmid by PCR; and,
- Direct sequencing of the insert sequence, with minimal sequence redundancy of three.

### Cell lines

The cell line used for initial testing is the 3T3-L1 Tet-Off which is a contact-inhibited, differentiable derivative of NIH/3T3 mouse fibroblasts (Clontech). 3T3-L1 Tet-Off cells is cultured as described by the provider.

These cells already express stably the appropriate regulatory tTA protein. This protein is responsible for the tightly regulated expression of the response gene. Gene expression in these cells is turned on when tetracycline or doxycycline (a tetracycline derivative) is removed from the culture medium.

Further cell lines are developed with the same technology, including HeLa, COS7 and CHO-K1, HepG2, Caco-2, MCF-7, HEK 293 and SW480 cells.

### Selection of double-stable transfected cells

Prior to establishing the double-stable Tet-Off 3T3 cell line, the functionality of the construct is tested by unstable transfection and Western blotting using a monoclonal antibody anti-T7 RNAP (Novagen).

Then, 3T3-L1 Tet-Off are cotransfected with the pTRE2-Hyg linear selection marker and the pBG-I vector containing T7 RNAP gene. Transfection is performed with Lipofectin reagent as described by the manufacturer (InVitrogen). Stably transfected cells are selected by Hygromycin, as described by the manufacturer (Clontech).

Because the expression and induction levels of the protein can be profoundly affected by the random site of integration, at least 30 clones are tested for basal and induced gene expression. Functionality is assayed by measuring β-galactosidase activity, then by Western-blotting against the T7 RNA polymerase (Novagen). The cell lines that give the highest overall induction and lowest uninduced expression level of T7 RNAP is used for further experiments.

### Production of knock-in mice expressing the T7 RNA polymerase

### Generation of the targeting vector

The targeting vector used for knock-in contains the two versions of the T7 RNAP gene (the wild type cytoplasmic *T7 RNAP*, plus the nuclear *T7 RNAP* containing a NLS) and *LacZ.* This vector is named T7RNAP^{nuc}/T7RNAP^{cyt}/LacZ targeting vector thereafter.

### Generation of knock-in mice

Embryonic stem (ES) clones having replaced one copy of the endogenous murine hypoxanthine phosphoribosyltransferase (HPRT) locus on the active X chromosome by the targeting vector are produced. The ES cells that are used derive from a highly mixed C57BL/6 and 129S1/SvlmJ genetic backgrounds. Transfection will be carried out by electroporation with the linearized T7RNAP^{nuc}/T7RNAP^{cyt}/LacZ targeting vector. Positive clones are selected by HAT medium (hypoxanthine, aminopterin and thymidine) given resistance conferred by HPRT. Positive clones are injected into C57BL/6 blastocysts, and reimplantation of injected blastocyts into uterus. This lead to chimeric offspring in nearly 100 % of cases with ∼100% germline transmission.

### Phenotype analysis

To ensure the absence of abnormal phenotype, the knock-in mice are examined for gross anomalies including general aspect, general behavior, autopsy analysis, and major blood features. The life expectancy of genetically engineered animals are also studied.

On the other hand, the expression of T7 RNAP and β-galactosidase are tested by Western-blotting and chemiluminescence as described above. The tissue required for analysis is obtained from homogenates of tail samples.

Then, mice are backcrossed into several backgrounds including C57BL/6J, 129S1/SvlmJ, BALB/cByJ et C3H/HeJ by a marker-assisted process (Markel, Shu et al. 1997).

### Assay of gene reporter expression, i.e. LacZ (β-galactosidase)

β-galactosidase activity is assayed as a reporter for:
- All the in vitro experiments
- The in vivo experiments that aim to investigate the level of gene inhibition across various organs.
β-galactosidase activity is measured by chemiluminescence using the Luminescent beta-galactosidase Reporter System 3 (Clontech). This assay is based on the cleavage of the substrate by beta-galactosidase that releases an unstable intermediate. This intermediate further degrades with the concurrent emission of light and provides a quantitative measure of beta-galactosidase activity.
Cell lysates (in vitro assays) and tissue homogenates (in vivo assays) are used to assay the activity of the gene reporter.

### Mouse insulin receptor (INSR) monitoring

Oligonucleotides targeting mouse *INSR* are used for all the in vivo experiments (except those that aim to investigate the level of gene inhibition across various organs). Gene inhibition is monitored by phenotypic analysis, i.e. the serum glucose level.

The complete inhibition of *INSR* expression causes a major change in the phenotype. For instance, non-conditional homozygous knock-out mice for *INSR* develop severe hyperglycemia and hyperketonemia within hours of birth, and die as the result of diabetic ketoacidosis in 48-72 hours. (Accili, Drago et al. 1996; Joshi, Lamothe et al. 1996). In contrast, heterozygous mice for *INSR* have no obvious phenotypic change.

### In vitro experimentation

Screening of oligonucleotide efficiency is performed in cell culture because mRNA structures in biological systems are likely to differ from the structure of in vitro transcribed RNA molecules, and because RNA-binding proteins shield certain target sites inside cells.

In vitro experimentations are carried out in a 24 or 96 well format cell culture plates. Cells are plated and grown at 60%-70% confluence, and then assayed as described below. Then, cells are harvested and β-galactosidase activity is measured as described previously.

### Selection of the most efficient antisense-like, ribozyme-like, siRNA-like and shRNA-like oligonucleotides targetihg β-galactosidase

The aim of this step is to select the oligonucleotides having the greatest inhibitory effect on β-galactosidase expression.
Other experimental conditions:
- oligonucleotide chemistry: phosphodiester
- Design of the T7 RNAP promoter sequence of the oligonucleotides: minimal promoter sequence, double stranded targeting sequence
- 3' modification: none
- Transfection agent: Lipofectin
- Time for assay: 0 hours and 24 hours.

### Selecting the most efficient chemically synthesized type of oligonucleotide

Two types of oligonucleotides are tested, i.e. phosphodiester and phosphorothiates oligonucleotides.
Other experimental conditions:
- Design of the T7 RNAP promoter sequence of the oligonucleotides: minimal promoter sequence, double stranded targeting sequence
- 3' modification: none
- Transfection agent: Lipofectin
- Time for assay: 0 hours and 24 hours
- All other condition selected as previously.

### Selection of optimal designs of the oligonucleotides

Eight different designs of the oligonucleotides (described above) are tested.
Other experimental conditions:
- 3' modification: none
- Transfection agent: Lipofectin
- Time for assay: 0 hours and 24 hours
- All other condition selected as previously.

### Selection of the optimal modification of the 3' extremities of oligonucleotides

Four different types of protection are compared, i.e. none, 3' inverted thymidine, 5-methyl cytosine at 3' extremities and circularization of oligonucleotides.
Other experimental conditions:
- Transfection agent: Lipofectin
- Time for assay: 0 hours and 24 hours
- All other condition selected as previously.

### Selection of the optimal conditions of cell transfection

Three transfection modalities are tested: none, Lipofectin and calcium-phosphate transfection.
Other experimental conditions:
- Time for assay: 0 hours and 24 hours
- All other condition selected as previously.

### Testing the duration of effect gene inhibition

The duration of effect is tested by serial analysis of gene inhibition after single cell treatment at 0 hours, 1 hour, 6 hours, 12 hours, 1 day, 2 days, 5 days, 10 days and 15 days
Other experimental conditions:
- All other condition selected as previously.

### In vivo experimentation

Knock-in mice are treated with antisense-like, ribozyme-like, siRNA-like, and shRNA-like oligonucleotides targeting *INSR*. Oligonucleotides are administrated once per day during 5 days, followed by 5-15 days period of wash-out. Then, the resulting changes of phenotype are assayed by measuring the serum glucose level every two days from day 0 to day 10.

### Selection of the most efficient type of oligonucleotides targeting INSR

The aim of this step is to select the oligonucleotides having the highest inhibition effect on *INSR* expression.
- oligonucleotide chemistry: the condition giving optimal results in vitro; if information lacking, phosphorothioate oligonucleotides are used
- Design of the oligonucleotides: the condition giving optimal results in vitro; if information lacking at this date, double stranded oligonucleotides with minimal promoter sequence are used
- 3' extremities modifications: the condition giving optimal inhibition results in vitro; if information lacking at this date, oligonucleotides with no 3' extremity modification are used
- Mode of administration: subcutaneous
- Dose of oligonucleotide: 50 mg/kg/injection.

### Selecting the most efficient type chemically synthesized oligonucleotide

Two types of oligonucleotides are tested, i.e. phosphodiester and phosphorothiates oligonucleotides.
- Design of the oligonucleotides: the condition giving optimal results in vitro; if information lacking at this date, double stranded oligonucleotides with minimal promoter sequence are used
- 3' extremities modifications: the condition giving optimal inhibition results in vitro; if information lacking at this date, oligonucleotides with no 3' extremity modification are used
- Mode of administration: subcutaneous
- Dose of oligonucleotide: 50 mg/kg/injection
- Other condition selected as previously.

### Selection of the optimal design of the oligonucleotides

Eight different designs of the oligonucleotides (as described above) are tested.
- 3' extremities modifications: the condition giving optimal inhibition results in vitro; if information lacking at this date, oligonucleotides with no 3' extremity modification are used
- Mode of administration: subcutaneous
- Dose of oligonucleotide: 50 mg/kg/injection
- All other conditions selected as previously.

### Selection of the optimal modification of the 3' extremities of oligonucleotides

Four different types of protection are compared, i.e. none, 3' inverted thymidine, 5-methyl cytosine at 3' extremities and circularization of oligonucleotides.
Other experimental conditions:
- Mode of administration: subcutaneous
- oligonucleotide dose: 50 mg/kg/injection
- All other conditions selected as previously.

### Selection of the optimal mode of oligonucleotide administration

Three different mode of administration are tested, i.e. subcutaneous, intravenous, and intraperitoneal administration.
Other experimental conditions:
- oligonucleotide dose: 50 mg/kg/injection
- All other conditions selected as previously.

### Selection of the optimal dose of oligonucleotide

Six different doses will be tested, i.e. 0, 10, 20, 30, 40 and 50 mg/kg/injection.
All other condition selected as previously

### REFERENCES

Abbas-Terki, T., W. Blanco-Bose, et al. (2002). "Lentiviral-mediated RNA interference." Hum Gene Ther **13**(18): 2197-201.

Abounader, R., B. Lal, et al. (2002). "In vivo targeting of SF/HGF and c-met expression via U1snRNA/ribozymes inhibits glioma growth and angiogenesis and promotes apoptosis." Faseb J **16**(1): 108-10.

Accili, D., J. Drago, et al. (1996). "Early neonatal death in mice homozygous for a null allele of the insulin receptor gene." Nat Genet **12**(1): 106-9.

Aigner, A., D. Fischer, et al. (2002). "Delivery of unmodified bioactive ribozymes by an RNA-stabilizing polyethylenimine (LMW-PEI) efficiently down-regulates gene expression." Gene Ther **9**(24): 1700-7.

Alt, M., R. Renz, et al. (1997). "Core specific antisense phosphorothioate oligodeoxynucleotides as potent and specific inhibitors of hepatitis C viral translation." Arch Virol **142**(3): 589-99.

An, D. S., Y. Xie, et al. (2003). "Efficient lentiviral vectors for short hairpin RNA delivery into human cells." Hum Gene Ther **14**(12): 1207-12.

Angell, S. M. and D. C. Baulcombe (1997). "Consistent gene silencing in transgenic plants expressing a replicating potato virus X RNA." Embo J **16**(12): 3675-84.

Aoki, M., R. Morishita, et al. (1997). "In vivo transfer efficiency of antisense oligonucleotides into the myocardium using HVJ-liposome method." Biochem Biophys Res Commun **231**(3): 540-5.

Astriab-Fisher, A., D. Sergueev, et al. (2002). "Conjugates of antisense oligonucleotides with the Tat and antennapedia cell-penetrating peptides: effects on cellular uptake, binding to target sequences, and biologic actions." Pharm Res **19**(6): 744-54.

Azhayeva, E., A. Azhayev, et al. (1997). "Inhibitory properties of double-helix-forming circular oligonucleotides." Nucleic Acids Res **25**(24): 4954-61.

Beltinger, C., H. U. Saragovi, et al. (1995). "Binding, uptake, and intracellular trafficking of phosphorothioate-modified oligodeoxynucleotides." J Clin Invest **95**(4): 1814-23.

Benton, B. M., W. K. Eng, et al. (1990). "Signal-mediated import of bacteriophage T7 RNA polymerase into the Saccharomyces cerevisiae nucleus and specific transcription of target genes." Mol Cell Biol **10**(1): 353-60.

Bernstein, E., A. A. Caudy, et al. (2001). "Role for a bidentate ribonuclease in the initiation step of RNA interference." Nature **409**(6818): 363-6.

Bohula, E. A., A. J. Salisbury, et al. (2003). "The efficacy of small interfering RNAs targeted to the type 1 insulin-like growth factor receptor (IGF1R) is influenced by secondary structure in the IGF1R transcript." J Biol Chem **278**(18): 15991-7.

Brisson, M., Y. He, et al. (1999). "A novel T7 RNA polymerase autogene for efficient cytoplasmic expression of target genes." Gene Ther **6**(2): 263-70.

Brummelkamp, T. R., R. Bernards, et al. (2002). "A system for stable expression of short interfering RNAs in mammalian cells." Science **296**(5567): 550-3.

Burgin, A. B. and N. R. Pace (1990). "Mapping the active site of ribonuclease P RNA using a substrate containing a photoaffinity agent." Embo J **9**(12): 4111-8.

Chaloin, L., P. Vidal, et al. (1998). "Design of carrier peptide-oligonucleotide conjugates with rapid membrane translocation and nuclear localization properties." Biochem Biophys Res Commun **243**(2): 601-8.

Chamberlin, M., J. McGrath, et al. (1970). "New RNA polymerase from Escherichia coli infected with bacteriophage T7." Nature **228**(268): 227-31.

Chen, W., S. Tabor, et al. (1987). "Distinguishing between mechanisms of eukaryotic transcriptional activation with bacteriophage T7 RNA polymerase." Cell **50**(7): 1047-55.

Chen, X., Y. Li, et al. (1995). "A novel nonviral cytoplasmic gene expression system and its implications in cancer gene therapy." Cancer Gene Ther **2**(4): 281-9.

Clarenc, J. P., B. Lebleu, et al. (1993). "Characterization of the nuclear binding sites of oligodeoxyribonucleotides and their analogs." J Biol Chem **268**(8): 5600-4.

Czubayko, F., A. M. Schulte, et al. (1996). "Melanoma angiogenesis and metastasis modulated by ribozyme targeting of the secreted growth factor pleiotrophin." Proc Natl Acad Sci U S A **93**(25): 14753-8.

Dagle, J. M., D. L. Weeks, et al. (1991). "Pathways of degradation and mechanism of action of antisense oligonucleotides in Xenopus laevis embryos." Antisense Res Dev **1**(1): 11-20.

Davanloo, P., A. H. Rosenberg, et al. (1984). "Cloning and expression of the gene for bacteriophage T7 RNA polymerase." Proc Natl Acad Sci U S A **81**(7): 2035-9.

Delano, J. P., J. E. Dombrowski, et al. (1999). "The expression of tomato prosystemin in Escherichia coli: A structural challenge." Protein Expr Purif **17**(1): 74-82.

Deuschle, U., R. Pepperkok, et al. (1989). "Regulated expression of foreign genes in mammalian cells under the control of coliphage T3 RNA polymerase and lac repressor." Proc Natl Acad Sci U S A **86**(14): 5400-4.

Dieci, G., L. Bottarelli, et al. (2000). "tRNA-assisted overproduction of eukaryotic ribosomal proteins." Protein Expr Purif **18**(3): 346-54.

Dominski, Z. and R. Kole (1994). "Identification and characterization by antisense oligonucleotides of exon and intron sequences required for splicing." Mol Cell Biol **14**(11): 7445-54.

Donze, O. and D. Picard (2002). "RNA interference in mammalian cells using siRNAs synthesized with T7 RNA polymerase." Nucleic Acids Res **30**(10): e46.

Doudna, J. A. and T. R. Cech (2002). "The chemical repertoire of natural ribozymes." Nature **418**(6894): 222-8.

Dower, K. and M. Rosbash (2002). "T7 RNA polymerase-directed transcripts are processed in yeast and link 3' end formation to mRNA nuclear export." Rna **8**(5): 686-97.

Dunn, J. J., B. Krippl, et al. (1988). "Targeting bacteriophage T7 RNA polymerase to the mammalian cell nucleus." Gene **68**(2): 259-66.

Elbashir, S. M., J. Harborth, et al. (2001). "Duplexes of 21-nucleotide RNAs mediate RNA interference in cultured mammalian cells." Nature **411**(6836): 494-8.

Elbashir, S. M., W. Lendeckel, et al. (2001). "RNA interference is mediated by 21- and 22-nucleotide RNAs." Genes Dev **15**(2): 188-200.

Elroy-Stein, O. and B. Moss (1990). "Cytoplasmic expression system based on constitutive synthesis of bacteriophage T7 RNA polymerase in mammalian cells." Proc Natl Acad Sci U S A **87**(17): 6743-7.

Fire, A., S. Xu, et al. (1998). "Potent and specific genetic interference by double-stranded RNA in Caenorhabditis elegans." Nature **391**(6669): 806-11.

Fisher, A. A., D. Ye, et al. (2002). "Evaluating the specificity of antisense oligonucleotide conjugates. A DNA array analysis." J Biol Chem **277**(25): 22980-4.

Fitzgerald, D. P. and W. Bender (2001). "Polycomb group repression reduces DNA accessibility." Mol Cell Biol **21**(19): 6585-97.

Fuerst, T. R., P. L. Earl, et al. (1987). "Use of a hybrid vaccinia virus-T7 RNA polymerase system for expression of target genes." Mol Cell Biol **7**(7): 2538-44.

Fuerst, T. R. and B. Moss (1989). "Structure and stability of mRNA synthesized by vaccinia virus-encoded bacteriophage T7 RNA polymerase in mammalian cells. Importance of the 5' untranslated leader." J Mol Biol **206**(2): 333-48.

Fuerst, T. R., E. G. Niles, et al. (1986). "Eukaryotic transient-expression system based on recombinant vaccinia virus that synthesizes bacteriophage T7 RNA polymerase." Proc Natl Acad Sci U S A **83**(21): 8122-6.

Gao, W. Y., C. Storm, et al. (1993). "Cellular pharmacology of phosphorothioate homooligodeoxynucleotides in human cells." Mol Pharmacol **43**(1): 45-50.

Gao, X. and L. Huang (1993). "Cytoplasmic expression of a reporter gene by co-delivery of T7 RNA polymerase and T7 promoter sequence with cationic liposomes." Nucleic Acids Res **21**(12): 2867-72.

Gewirtz, A. M., D. L. Sokol, et al. (1998). "Nucleic acid therapeutics: state of the art and future prospects." Blood **92**(3): 712-36.

Golomb, M. and M. Chamberlin (1974). "Characterization of T7-specific ribonucleic acid polymerase. IV. Resolution of the major in vitro transcripts by gel electrophoresis." J Biol Chem **249**(9): 2858-63.

Gossen, M. and H. Bujard (1992). "Tight control of gene expression in mammalian cells by tetracycline-responsive promoters." Proc Natl Acad Sci U S A **89**(12): 5547-51.

Gossen, M., S. Freundlieb, et al. (1995). "Transcriptional activation by tetracyclines in mammalian cells." Science **268**(5218): 1766-9.

Grishok, A., A. E. Pasquinelli, et al. (2001). "Genes and mechanisms related to RNA interference regulate expression of the small temporal RNAs that control C. elegans developmental timing." Cell **106**(1): 23-34.

Hamilton, A. J. and D. C. Baulcombe (1999). "A species of small antisense RNA in posttranscriptional gene silencing in plants." Science **286**(5441): 950-2.

Hannon, G. J. (2002). "RNA interference." Nature **418**(6894): 244-51.

Hoffman, L. M. and M. G. Johnson (1994). "Enzymatic synthesis of milligram quantities of ribozymes in small volumes." Biotechniques **17**(2): 372-5.

Hofker, M. and J. W. Voncken (2002). Transgenic Mouse Methods and Protocols. Oxford, U.K., Blackwell Publishing.

Holt, C. A. and R. N. Beachy (1991). "In vivo complementation of infectious transcripts from mutant tobacco mosaic virus cDNAs in transgenic plants." Virology **181**(1): 109-17.

Johnson, K. L. and L. A. Ball (1999). "Induction and Maintenance of Autonomous Flock House Virus RNA1 Replication." J. Virol. **73**(10): 7933-7942.

Joshi, R. L., B. Lamothe, et al. (1996). "Targeted disruption of the insulin receptor gene in the mouse results in neonatal lethality." Embo J **15**(7): 1542-7.

Katoh, T., M. Susa, et al. (2003). "Simple and rapid synthesis of siRNA derived from in vitro transcribed shRNA." Nucleic Acids Res Suppl(3): 249-50.

Klein, C., C. T. Bock, et al. (2003). "Inhibition of hepatitis B virus replication in vivo by nucleoside analogues and siRNA." Gastroenterology **125**(1): 9-18.

Kochetkov, S. N., E. E. Rusakova, et al. (1998). "Recent studies of T7 RNA polymerase mechanism." FEBS Lett **440**(3): 264-7.

Koken, M. H., H. H. Odijk, et al. (1993). "Augmentation of protein production by a combination of the T7 RNA polymerase system and ubiquitin fusion: overproduction of the human DNA repair protein, ERCC1, as a ubiquitin fusion protein in Escherichia coli." Biochem Biophys Res Commun **195**(2): 643-53.

Kollar, A., T. Dalmay, et al. (1993). "Defective interfering RNA-mediated resistance against cymbidium ringspot tombusvirus in transgenic plants." Virology **193**(1): 313-8.

Kurreck, J. (2003). "Antisense technologies. Improvement through novel chemical modifications." Eur J Biochem **270**(8): 1628-44.

Kurreck, J., E. Wyszko, et al. (2002). "Design of antisense oligonucleotides stabilized by locked nucleic acids." Nucleic Acids Res **30**(9): 1911-8.

Lee, N. S., T. Dohjima, et al. (2002). "Expression of small interfering RNAs targeted against HIV-1 rev transcripts in human cells." Nat Biotechnol **20**(5): 500-5.

Lee, P. A., L. M. Blatt, et al. (2000). "Pharmacokinetics and tissue distribution of a ribozyme directed against hepatitis C virus RNA following subcutaneous or intravenous administration in mice." Hepatology **32**(3): 640-6.

Lewis, D. L., J. E. Hagstrom, et al. (2002). "Efficient delivery of siRNA for inhibition of gene expression in postnatal mice." Nat Genet **32**(1): 107-8.

Li, K., S. Y. Lin, et al. (2003). "Use of RNA interference to target cyclin E-overexpressing hepatocellular carcinoma." Cancer Res **63**(13): 3593-7.

Lieber, A., U. Kiessling, et al. (1989). "High level gene expression in mammalian cells by a nuclear T7-phage RNA polymerase." Nucleic Acids Res **17**(21): 8485-93.

Lipardi, C., Q. Wei, et al. (2001). "RNAi as random degradative PCR: siRNA primers convert mRNA into dsRNAs that are degraded to generate new siRNAs." Cell **107**(3): 297-307.

Liu, D., T. Ren, et al. (2003). "Cationic transfection lipids

Cationic transfection lipids in gene therapy: successes, set-backs, challenges and promises

### Cationic lipids for transfection

Structure-activity relationship in cationic lipid mediated gene transfection." Curr Med Chem **10**(14): 1307-15.

Lohmann, J. U., I. Endl, et al. (1999). "Silencing of developmental genes in Hydra." Dev Biol **214**(1): 211-4.

Lyakhov, D. L., B. He, et al. (1997). "Mutant bacteriophage T7 RNA polymerases with altered termination properties." J Mol Biol **269**(1): 28-40.

Magee, A. M. and T. A. Kavanagh (2002). "Plastid genes transcribed by the nucleus-encoded plastid RNA polymerase show increased transcript accumulation in transgenic plants expressing a chloroplast-localized phage T7 RNA polymerase." J Exp Bot **53**(379): 2341-9.

Makkerh, J. P., C. Dingwall, et al. (1996). "Comparative mutagenesis of nuclear localization signals reveals the importance of neutral and acidic amino acids." Curr Biol **6**(8): 1025-7.

Markel, P., P. Shu, et al. (1997). "Theoretical and empirical issues for marker-assisted breeding of congenic mouse strains." Nat Genet **17**(3): 280-4.

McCaffrey, A. P., L. Meuse, et al. (2003). "A potent and specific morpholino antisense inhibitor of hepatitis C translation in mice." Hepatology **38**(2): 503-8.

McCaffrey, A. P., H. Nakai, et al. (2003). "Inhibition of hepatitis B virus in mice by RNA interference." Nat Biotechnol **21**(6): 639-44.

McManus, M. T. and P. A. Sharp (2002). "Gene silencing in mammals by small interfering RNAs." Nat Rev Genet **3**(10): 737-47.

Miller, P. S. (1991). "Oligonucleoside methylphosphonates as antisense reagents." Biotechnology (N Y) **9**(4): 358-62.

Milligan, J. F., D. R. Groebe, et al. (1987). "Oligoribonucleotide synthesis using T7 RNA polymerase and synthetic DNA templates." Nucleic Acids Res **15**(21): 8783-98.

Milligan, J. F. and O. C. Uhlenbeck (1989). "Synthesis of small RNAs using T7 RNA polymerase." Methods Enzymol **180**: 51-62.

Misquitta, L. and B. M. Paterson (1999). "Targeted disruption of gene function in Drosophila by RNA interference (RNA-i): a role for nautilus in embryonic somatic muscle formation." Proc Natl Acad Sci U S A **96**(4): 1451-6.

Nakano, R., T. Nakagawa, et al. (2003). "A novel T7 system utilizing mRNA coding for T7 RNA polymerase." Biochem Biophys Res Commun **301**(4): 974-8.

Ngo, H., C. Tschudi, et al. (1998). "Double-stranded RNA induces mRNA degradation in Trypanosoma brucei." Proc Natl Acad Sci U S A **95**(25): 14687-92.

Nicolazzi, C., M. Garinot, et al. (2003). "Cationic lipids for transfection." Curr Med Chem **10**(14): 1263-77.

Oakley, J. L. and J. E. Coleman (1977). "Structure of a promoter for T7 RNA polymerase." Proc Natl Acad Sci U S A **74**(10): 4266-70.

Oehlke, J., P. Birth, et al. (2002). "Cellular uptake of antisense oligonucleotides after complexing or conjugation with cell-penetrating model peptides." Eur J Biochem **269**(16): 4025-32.

Opalinska, J. B. and A. M. Gewirtz (2002). "Nucleic-acid therapeutics: basic principles and recent applications." Nat Rev Drug Discov **1**(7): 503-14.

Paddison, P. J., A. A. Caudy, et al. (2002). "Short hairpin RNAs (shRNAs) induce sequence-specific silencing in mammalian cells." Genes Dev **16**(8): 948-58.

Paddison, P. J., A. A. Caudy, et al. (2002). "Stable suppression of gene expression by RNAi in mammalian cells." Proc Natl Acad Sci U S A **99**(3): 1443-8.

Romano, N. and G. Macino (1992). "Quelling: transient inactivation of gene expression in Neurospora crassa by transformation with homologous sequences." Mol Microbiol **6**(22): 3343-53.

Ruiz, M. T., O. Voinnet, et al. (1998). "Initiation and maintenance of virus-induced gene silencing." Plant Cell **10**(6): 937-46.

Salehi-Ashtiani, K. and J. W. Szostak (2001). "In vitro evolution suggests multiple origins for the hammerhead ribozyme." Nature **414**(6859): 82-4.

Sambrook, J., E. F. Fritsch, et al. (1989). Molecular Cloning, a laboratory manual. N.Y., Cold Spring Harbor Laboratory Press.

Sanchez Alvarado, A. and P. A. Newmark (1999). "Double-stranded RNA specifically disrupts gene expression during planarian regeneration." Proc Natl Acad Sci U S A **96**(9): 5049-54.

Scherr, M., K. Battmer, et al. (2003). "Modulation of gene expression by lentiviral-mediated delivery of small interfering RNA." Cell Cycle **2**(3): 251-7.

Seksek, O. and J. Bolard (1996). "Nuclear pH gradient in mammalian cells revealed by laser microspectrofluorimetry." J Cell Sci **109**(Pt 1): 257-62.

Shakin, S. H. and S. A. Liebhaber (1986). "Destabilization of messenger RNA/complementary DNA duplexes by the elongating 80 S ribosome." J Biol Chem **261**(34): 16018-25.

Song, E., S. K. Lee, et al. (2003). "RNA interference targeting Fas protects mice from fulminant hepatitis." Nat Med **9**(3): 347-51.

Stein, C. A., J. L. Tonkinson, et al. (1993). "Dynamics of the internalization of phosphodiester oligodeoxynucleotides in HL60 cells." Biochemistry **32**(18): 4855-61.

Summers, W. C. and R. B. Siegel (1970). "Transcription of late phage RNA by T7 RNA polymerase." Nature **228**(277): 1160-2.

Tari, A. M., M. Andreeff, et al. (1996). "Cellular uptake and localization of liposomal-methylphosphonate oligodeoxynucleotides." J Mol Med **74**(10): 623-8.

Timmons, L. and A. Fire (1998). "Specific interference by ingested dsRNA." Nature **395**(6705): 854.

Tiranti, V., A. Savoia, et al. (1997). "Identification of the gene encoding the human mitochondrial RNA polymerase (h-mtRPOL) by cyberscreening of the Expressed Sequence Tags database." Hum Mol Genet **6**(4): 615-25.

Tung, C. H., J. Wang, et al. (1995). "Dual-specificity interaction of HIV-1 TAR RNA with Tat peptide-oligonucleotide conjugates." Bioconjug Chem **6**(3): 292-5.

Tunitskaya, V. L. and S. N. Kochetkov (2002). "Structural-functional analysis of bacteriophage T7 RNA polymerase." Biochemistry (Mosc) **67**(10): 1124-35.

Usman, N. and L. M. Blatt (2000). "Nuclease-resistant synthetic ribozymes: developing a new class of therapeutics." J Clin Invest **106**(10): 1197-202.

Verri, T., F. Argenton, et al. (1997). "The bacteriophage T7 binary system activates transient transgene expression in zebrafish (Danio rerio) embryos." Biochem Biophys Res Commun **237**(3): 492-5.

Vickers, T. A., S. Koo, et al. (2003). "Efficient reduction of target RNAs by small interfering RNA and RNase H-dependent antisense agents. A comparative analysis." J Biol Chem **278**(9): 7108-18.

Wargelius, A., S. Ellingsen, et al. (1999). "Double-stranded RNA induces specific developmental defects in zebrafish embryos." Biochem Biophys Res Commun **263**(1): 156-61.

Wirtz, E., C. Hartmann, et al. (1994). "Gene expression mediated by bacteriophage T3 and T7 RNA polymerases in transgenic trypanosomes." Nucleic Acids Res **22**(19): 3887-94.

Wirtz, E., M. Hoek, et al. (1998). "Regulated processive transcription of chromatin by T7 RNA polymerase in Trypanosoma brucei." Nucleic Acids Res **26**(20): 4626-34.

Yarovoi, S. V. and T. Pederson (2001). "Human cell lines expressing hormone regulated T7 RNA polymerase localized at distinct intranuclear sites." Gene **275**(1): 73-81.

Yu, J. Y., S. L. DeRuiter, et al. (2002). "RNA interference by expression of short-interfering RNAs and hairpin RNAs in mammalian cells." Proc Natl Acad Sci U S A **99**(9): 6047-52.

Zamore, P. D., T. Tuschl, et al. (2000). "RNAi: double-stranded RNA directs the ATP-dependent cleavage of mRNA at 21 to 23 nucleotide intervals." Cell **101**(1): 25-33.

## Claims

1. A method for inhibiting the expression of a targeted gene in a cell or a non-human organism expressing a single-subunit RNA polymerase, comprising the following steps:
- providing a molecule comprising a sequence encoding an expression-inhibiting oligonucleotide specific for said targeted gene, operably linked to element(s) allowing transcription of said sequence by the single-subunit RNA polymerase in said cell or said non-human organism;
- introducing said molecule into said cell or non-human organism so that said sequence is expressed in said cell or non-human organism, thereby inhibiting the expression of said targeted gene.

2. A method for identifying or studying the function of a gene in a cell or a non-human organism expressing a single-subunit RNA polymerase, comprising the following steps:
- providing a molecule comprising a sequence encoding an expression-inhibiting oligonucleotide specific for said gene, operably linked to element(s) allowing transcription of said sequence by the single-subunit RNA polymerase in said cell or said non-human organism;
- introducing said molecule into said cell or non-human organism so that said sequence is expressed in said cell or non-human organism, thereby inhibiting the expression of said gene; and,
- determining the effect of the inhibition of expression of said gene on said cell or organism.

3. A method for preparing a cell or a non-human organism with an inhibited expression of a targeted gene, comprising the steps of:
- providing a cell or a non-human organism expressing a single-subunit RNA polymerase;
- providing a molecule comprising a sequence encoding an expression-inhibiting oligonucleotide specific for said targeted gene, operably linked to element(s) allowing transcription of said sequence by the single-subunit RNA polymerase in said cell or said organism; and,
- introducing said molecule into said cell or non-human organism so that said sequence is expressed in said cell or non-human organism, thereby inhibiting the expression of said targeted gene.

4. The method according to any one of claims 1-3, wherein the cell or the non-human organism is a recombinant cell or a non-human transgenic organism expressing a single-subunit RNA polymerase.

5. A kit comprising 1) a cell or a non-human organism expressing a single-subunit RNA polymerase, and, 2) a molecule comprising a sequence encoding an expression-inhibiting oligonucleotide specific for a targeted gene, operably linked to element(s) allowing transcription of said sequence by the single-subunit RNA polymerase in said recombinant cell or said non-human transgenic organism.

6. Use of a non-human transgenic organism expressing a single-subunit RNA polymerase in combination with a molecule comprising a sequence encoding an expression-inhibiting oligonucleotide specific for a targeted gene, operably linked to element(s) allowing transcription of said sequence by said single-subunit RNA polymerase for inhibiting the expression of said targeted gene or for identifying/studying the function of said targeted gene.

7. The method according to any one of claims 1-4 or the kit according to claim 5 or the use according to claim 6, wherein said single-subunit RNA polymerase is a bacteriophage RNA polymerase.

8. The method, the kit or the use according to claim 7, wherein said bacteriophage RNA polymerase is selected from the group consisting of T7 RNA polymerase, T3 RNA polymerase, SP6 RNA polymerase, and K11 RNA polymerase.

9. The method, the kit or the use according to claim 8, wherein said bacteriophage RNA polymerase is T7 RNA polymerase.

10. The method according to any one of claims 1-4 or the kit according to claim 5 or the use according to claim 6, wherein said single-subunit RNA polymerase is a mitochondrial RNA polymerase.

11. The method according to any one of claims 1-4 or the kit according to claim 5 or the use according to claim 6, wherein said single-subunit RNA polymerase is a chloroplast RNA polymerase.

12. The method, the kit or the use according to any one of claims 7-10, wherein said expression-inhibiting oligonucleotide is selected from the group consisting of an antisense RNA, a siRNA, a shRNA and a ribozyme.

13. The method, the kit or the use according to any one of claims 7-12, wherein said element(s) allowing transcription comprise the minimal promoter of said single-subunit RNA polymerase.

14. The method, the kit or the use according to claim 13, wherein said minimal promoter comprises the promoter sequence of nucleotides -17 through +1 of said single subunit RNA polymerase.

15. The method, the kit or the use according to any one of claims 7-14, wherein said recombinant cell is an eukaryotic cell.

16. The method, the kit or the use according to claim 15, wherein said eukaryotic cell is a mammalian cell.

17. The method, the kit or the use according to any one of claims 7-14, wherein said non-human transgenic organism is a non-human mammal.

18. The method, the kit or the use according to claim 17, wherein said non-human mammal is a mouse.

19. A non-human transgenic mammal expressing a heterologous single-subunit RNA polymerase, wherein an expression cassette encoding said single-subunit RNA polymerase is integrated in the genome of said non-human transgenic mammal.

20. The non-human transgenic mammal according to claim 19, wherein said single-subunit RNA polymerase is a bacteriophage RNA polymerase.

21. The non-human transgenic mammal according to claim 20, wherein said bacteriophage RNA polymerase is selected from the group consisting of T7 RNA polymerase, T3 RNA polymerase, SP6 RNA polymerase, and K11 RNA polymerase.

22. The non-human transgenic mammal according to claim 21, wherein said bacteriophage RNA polymerase is T7 RNA polymerase.

23. The non-human transgenic mammal according to claim 19, wherein said single-subunit RNA polymerase is a mitochondrial RNA polymerase.

24. The non-human transgenic mammal according to claim 19, wherein said single-subunit RNA polymerase is a chloroplast RNA polymerase.

25. The non-human transgenic mammal according to claim 19-24, wherein said non-human mammal is a mouse.

26. Use of a non-human transgenic mammal according any one of claims 19-25 for inhibing the expression of a targeted gene or for identifying/studying the function of a targeted gene.

27. A molecule comprising a sequence encoding an expression-inhibiting oligonucleotide operably linked to element(s) allowing transcription of said sequence by a single-subunit RNA polymerase, wherein said molecule has a structure selected from the group consisting of:
- a hairpin oligonucleotide comprising a minimal double-stranded RNA polymerase promoter sequence operably linked to a single-stranded oligonucleotide comprising the sequence encoding the expression-inhibiting oligonucleotide; and,
- a hairpin oligonucleotide comprising a minimal double-stranded RNA polymerase promoter sequence operably linked to a double-stranded oligonucleotide comprising the sequence encoding the expression-inhibiting oligonucleotide.
